# EUROPEAN PATENT SPECIFICATION

(11) **EP 4 090 263 B1**
(45) Date of publication and mention of the grant of the patent: **24.06.2026**
(21) Application number: 21741919.1
(22) Date of filing: 12.01.2021
(51) Int. Cl.: A61B 17/135, A63B 69/00

(54) **BLOOD FLOW RESTRICTION SYSTEMS HAVING WIRELESS MONITORING AND CONTROL**
BLUTFLUSSBEGRENZUNGSSYSTEME MIT DRAHTLOSER ÜBERWACHUNG UND STEUERUNG
SYSTÈMES DE RESTRICTION DE FLUX SANGUIN AYANT UNE SURVEILLANCE ET UNE COMMANDE SANS FIL

(30) Priority: 14.01.2020 US 202062960957 P; 27.01.2020 US 202016773102
(43) Date of publication of application: 23.11.2022
(73) Proprietor: Smart Tools Plus, LLC, Strongsville, Ohio 44149 (US)
(72) Inventor: COLOSI, Nicholas, Strongsville, Ohio 44149 (US)
(74) Representative: Grosse Schumacher Knauer von Hirschhausen
(86) International application number: PCT/US2021/013029
(87) International publication number: WO 2021/146158

(56) References cited:
- WO-A1-2019/068147
- WO-A1-2019/068147
- US-A1- 2010 324 429
- US-A1- 2011 160 022
- US-A1- 2017 112 504
- US-A1- 2019 133 215
- US-B1- 9 877 733

## Description

The present disclosure claims priority on United States Provisional Application No. 62/960,957 filed January 14, 2020, which herein referenced.

The present disclosure is also a continuation-in-part of United States Application No. 16/773,102 filed January 27, 2020, which in turn claims priority on United States Provisional Application No. 62/797,689 filed January 28, 2019, which are herein referenced.

### FIELD OF THE DISCLOSURE

This disclosure relates a method for exercising using a blood flow restriction training (BFRT) system and to a blood flow restriction training (BFRT) system according to the preamble of claims 1 and 8 respectively 10, and thus more generally to blood flow restriction systems, specifically to a blood flow restriction system that can be wirelessly monitored and/or controlled, more specifically to a blood flow restriction system that can be wirelessly monitored and/or controlled to a specific percentage of limb occlusion pressure (LOP), and even more specifically to a blood flow restriction system that can be wirelessly monitored and/or controlled to measure the LOP for a particular limb of a user and then set the inflatable belt system to a specific percentage of LOP as set by the user.

### BACKGROUND

Blood Flow Restriction Training (BFRT) is becoming a popular tool to improve muscle strength, size, and functional aerobic capacity in shorter amounts of time with less stress on the body than typical training. Practitioners began using BFRT in the treatment and recovery from musculoskeletal injuries or disabilities. Trainers and coaches have used BFRT as an adjunct for a usual training regimen or as a tool to aid recovery. BFRT is the brief and intermittent occlusion or restriction of arterial and venous blood flow performed by applying a tourniquet to the upper or lower extremity. BFRT has been found to augment skeletal muscle adaptation, along with systemic whole body changes and cardiovascular benefits while at rest, with low intensity endurance exercises or low load resistance training. BFRT has been found to be safe when applied with pressures relative to the cuff width and individual limb circumference which is obtained through measuring limb occlusion pressures via a Doppler.

BFRT is done by wrapping a tourniquet around the top portion of one's upper or lower limbs. The wrapping restricts blood flow from the veins of the working muscles to the heart and limits the amount of blood flow to the limbs from the arteries. The restriction results in physiological changes that mimic changes associated with high intensity exercise. The results are gains in muscle size and strength and increases in cardiovascular function at much lower intensities than are usually required for adaptation.

Various types of BFRT cuffs have been developed as described in US Patent Nos. 6,149,618; 7,413,527; 7,455,630; 8,021,283; 8,182,403; 8,273,114; 8,328,693; 8,366,740; 8,425,426; 8,992,397; 9,301,701; and 10,245,458; US Publication Nos. US 2009/0124912; 2015/0150560; US 2016/0193491; US 2017/0112504; US 2017/0224357; 2017/0325825; and US 2018/0290005; PCT Publication Nos. WO 2016/087123; WO 2017/149690; and WO 2019/068147; and the Smart Cuff^{™} devices offered by Smart Tools Plus, LLC (https://www.smarttoolsplus.com/), all of which are herein referenced. US 2019/0133215 A1 discloses a pneumatic training device and garment for increasing strength using an example pressure from a previous session, a predictive algorithm or the pressure may be determined while a compression means is on a user. WO 2019/068147 A1 discloses a blood occlusion or restriction cuff using a smart device.

Although these prior art BFRT cuffs can be successfully used for BFRT, these prior art BFRT cuffs typically require manual inflation/deflation, use of manual pressure gauges to monitor the pressure of the cuff, and the inconvenient adjustment of the cuff pressure during training. As such, there remains a need for a BFRT cuff having improved convenience of use during BFRT.

### SUMMARY

The invention provides a method for exercising using a blood flow restriction training (BFRT) system, and a blood flow restriction training (BFRT) system, according to claims 1, 8 and 10. The BFRT system in accordance with the present disclosure is designed/configured to address the continued needs of individuals using BFRT. The BFRT system of the present disclosure includes and/or is used with one or more of the following features:
- a BFRT system that is simple and inexpensive to manufacture;
- a BFRT system that is comfortable for use by the user;
- a BFRT system that is simple to use by the user;
- a BFRT system that is easy to apply and remove by the user;
- a BFRT system that is compact, lightweight, and easy to use, transport and store;
- a BFRT system that is can be used for a wide range of user body types;
- a BFRT system that is washable;
- a BFRT system that is durable;
- a BFRT system including contours to the body when inflated;
- a BFRT system including one or more air chambers;
- a BFRT system that is at least 11 inches long (e.g., 11-30 inches);
- a BFRT system having a urethane-coated air bladder;
- a BFRT system including a motor/pump unit to inflate/deflate the one or more air chambers in the BFRT system;
- a BFRT system including a motor/pump unit to inflate/deflate the one or more air chambers during use of the BFRT system;
- a BFRT system including a motor/pump unit that is detachable/connectable to the BFRT cuff and can remain connected to the BFRT cuff or be removed from the BFRT cuff prior to, during, and/or after use of the BFRT cuff;
- a BFRT system including a battery-powered motor/pump unit;
- a BFRT system including a battery-powered motor/pump unit wherein the battery is rechargeable;
- a BFRT system including a motor/pump unit having one or more displays regarding a) whether the pump is on/off, b) battery status, c) pressure level for pressure in the one or more air chambers of the BFRT cuff, d) time/date, e) whether pressure in the one of more air chambers of the BFRT cuff is at or below limb occlusion pressure, f) error notification/status, g) security status (e.g., locked, unlocked, etc.), h) ambient temperature, i) timer/clock information, j) alarm status/notification, k) time of use, l) pressure mode, operation mode and/or selected training session, m) whether full occlusion has been obtained, n) whether maximum workout time period has occurred, o) wireless connection status to computer, tablet, smart phone, and/or other type of smart device. p) preset value for % LOP, and/or q) preset % value for % LOP;
- a BFRT system including a motor/pump unit that can be manually controlled on the motor/pump unit itself and/or be controlled wired and/or wirelessly by a computer, tablet, smart phone, and/or other type of smart device;
- a BFRT system including a motor/pump unit wherein the air pressure in the one or more air chambers can be manually controlled on the motor/pump unit itself and/or be controlled wire and/or wirelessly by a computer, tablet, smart phone, and/or other type of smart device using a computer program or mobile app;
- a BFRT system including a motor/pump unit wherein the air pressure in the one or more air chambers can be automatically adjusted for pressure changes during use of the BFRT cuff;
- a BFRT system including a motor/pump unit that can remain on the BFRT cuff during exercise or be removed from the BFRT cuff prior to, during, or after the user exercises;
- a BFRT system including a pressure sensor and one or more processors to a) facilitate in controlling/maintaining/regulating the pressure in the one or more air chambers of the BFRT cuff, b) provide pressure information on the housing of the motor/pump unit itself or on some other region of the BFRT cuff, and/or c) provide pressure information wired and/or wirelessly to a computer, tablet, smart phone, and/or other type of smart device and have such information displayed using a computer program or mobile app;
- a BFRT system that maintains the pressure in the one or more air chambers of the BFRT cuff during use of the BFRT cuff by a user;
- a BFRT system that can be manually or automatically operated;
- a BFRT system allowing for manual input of a predetermined limb occlusion pressure and using such information to control/maintain/regulate the pressure in the one or more air chambers of the BFRT cuff;
- a BFRT system including wired and/or wirelessly receives information regarding the automatic calculation of a predetermined limb occlusion pressure via a computer program or mobile app running on a computer, tablet, smart phone, and/or other type of smart device and using such information to control/maintain/regulate the pressure in the one or more air chambers of the BFRT cuff;
- a BFRT system including a sensor to automatically shut off the pump and/or release pressure in the one or more air chambers of the BFRT cuff after sensing that an occlusion pressure in the one or more air chambers has been maintained for some preset period of time (e.g., up to 30 seconds, up to 1 minute, up to 4 minutes, up to 5 minutes, 0.001 seconds to 8 minutes [and all values and ranges therebetween], etc.);
- a BFRT system including a sensor to automatically shut off the pump and/or releases pressure in the one or more air chambers of the BFRT cuff after some preset period of time (e.g., 20 minutes, 5 seconds to 30 minutes [and all values and ranges therebetween], etc.);
- a BFRT system including a sensor to automatically shut off the pump and/or releases pressure in the one or more air chambers of the BFRT cuff after some preset period of time of non-use of the BFRT cuff (e.g., 10 minutes, 5 seconds to 30 minutes [and all values and ranges therebetween], etc.);
- a BFRT system including a GPS sensor or other type of location sensor;
- a BFRT system including one or more movement sensors;
- a BFRT system including a gyroscope sensor;
- a BFRT system including one or more pressure modes (e.g., blood flow restriction mode, ischemic preconditioning mode, manual mode, auto mode, etc.);
- a BFRT system that wired and/or wirelessly receives information used to control operation of the BFRT cuff;
- a BFRT system that wired and/or wirelessly receives information used to select the mode of operation of the BFRT system (e.g., ischemic preconditioning mode ("IPC"), manual mode, auto mode, etc.);
- a BFRT system including a detachable motor/pump unit;
- a BFRT system including a pressure port to facilitate in pressure maintenance in the one or more air chambers of the BFRT cuff once the motor/pump unit is detached from the BFRT cuff;
- a BFRT system including one or more pressure sensors to control one or more valves to the one or more air chambers in the BFRT cuff to automatically reduce pressure to a set or predetermined pressure for the one or more air chambers in the BFRT cuff;
- a BFRT system including a control system for auto-regulation of pressure in the BFRT cuff during use of the BFRT cuff by a user;
- a BFRT system including a control system for auto-regulation of pressure in the BFRT cuff within a certain pressure range of a set pressure or set % LOP during use of the BFRT cuff by a user;
- a BFRT system including a control system to partially or fully deflate the pressure in the BFRT cuff after the user completes a rep or exercise routine, and then reinflates the BFRT cuff when the user begins a new rep or exercise;
- a BFRT system including a control system to partially or fully deflate the pressure in the BFRT cuff after the BFRT cuff immediately detects or detects after a period of time that the user has stopped using the BFRT cuff, and optionally reinflates that BFRT cuff when the BFRT cuff detects use of the BFRT cuff by the user;
- a BFRT system including one or more pressure sensors to control one or more valves to the one or more air chambers in the BFRT cuff to automatically reduce pressure to a set or predetermined pressure for the one or more air chambers in the BFRT cuff;
- a computer program or mobile app that can run on a computer, server, cloud, tablet, smart phone, and/or other type of smart device that is used to select a BFRT training session, keep track of BFRT training sessions, provide suggested training tips for BFRT, provide suggested BFRT sessions, etc.; and/or
- a computer program or mobile app that can run on a computer, server, cloud, tablet, smart phone, and/or other type of smart device that uses password and/or other security controls to prevent unauthorized use of the BFRT system and/or access to personal information regarding the use of the BFRT system.

In another and/or alternative non-limiting aspect of the present disclosure, there is provided a BFRT system including a motor/pump unit that includes a) a housing; b) a motor; c) a pump; d) one or more displays (e.g., LED screen, OLED screen, etc.) to display measured pressure and/or to display other information; e) optional one or more processors; f) wireless electronics to send/receive wireless signals between the motor/pump unit and a computer, server, data hub, cloud, tablet, smart phone and/or other type of smart device; g) one or more pressure gauges and/or sensors used to measure a gas pressure in the air inflatable system; h) optional power source to power one or more components of the motor/pump unit (e.g., motor, pump, pressure sensor/gauge; electronics, display, etc.); i) optional one or more power ports to charge the power source; j) optional connection arrangement to permanently or releasably connect the motor/pump unit to the outer material cover; k) optional one or more fluid connectors to fluidly connect the motor/pump unit to the air inflatable system; l) optional GPS system; m) optional circuit board and other electronics; n) optional software; o) optional one or more data ports (e.g., micro USB port, etc.) to enable wired transfer of data between the motor/pump unit and a computer, server, data hub, cloud, tablet, smart phone and/or other type of smart device; p) optional one or more buttons (e.g., power button, etc.), q) optional one or more selection or scroll buttons to enable manual operation/control of the motor/pump unit and/or to select certain information to be displayed on the motor/pump unit; q) optional one or more switch(es) to enable manual operation/control of the motor/pump unit; r) optional air hose port; s) optional one or more lights and/or other indicators to indicate a mode and/or operation and/or status of the motor/pump unit; t) optional memory; u) optional gyroscope system to measure/determine/detect movement and/or movement direction of the BFRT cuff during use of the BFRT cuff by a user; and/or v) one or more movement/motion sensors to measure/determine/detect movement and/or movement direction of the BFRT cuff during use of the BFRT cuff by a user.

In another and/or alternative non-limiting aspect of the present invention, the motor/pump unit can be sized and have a weight that can be easily handled by a user. Generally, the volume of the housing of the motor/pump unit is no more than 8.19 × 10³ cm³ (500 cubic inches), typically 8.19-8.19 × 10³ cm³ (0.5-500 cubic inches) (and all values and ranges therebetween), and more typically 16.39-819.35 cm³ (1-50 cubic inches). Generally, the weight of the motor/pump unit, including the housing and optional air hose, is no more than 4.54 kg (10 lbs), typically 0.0227-4.54 kg (0.05-10 lbs) (and all values and ranges therebetween), and more typically 0.0907-1.36 kg (0.2-3 lbs). Generally, the size and shape of the housing is such that it can be held and carried above a ground surface in a single hand of an average adult user without need of any further assistance or support system. In one specific non-limiting embodiment, the motor/pump unit has a volume of less than 491.6 cm³ (30 cubic inches) and a weight of less than 0.680 kg (1.5 lbs).

In another and/or alternative non-limiting aspect of the present disclosure, there is provided a BFRT cuff including one or more air bladders that can be inflated and deflated and a detachable air pump configured to inflated and deflate the one or more air bladders of the BFRT cuff. The BFRT cuff is generally formed of a flexible material (e.g., nylon material, neoprene, Kevlar^{™}, etc.) to partially or fully house the one or more air bladders. The one or more air bladders can be formed of a rubber material or any other material that can be inflated and retain air within the air bladder until deflated. The length of the BFRT cuff is generally 20-70 cm (and all values and ranges therebetween) for use about an arm and generally 30-100 cm (and all values and ranges therebetween) for use about a leg; however, other lengths can be used. The width of the BFRT cuff is at least 5 cm, typically about 5-14 cm (and all values and ranges therebetween), and more typically about 8-12 cm; however, other widths can be used. The BFRT cuff includes one or more air connection arrangements to inflate/deflate the one or more air bladders. Generally, the one or more air connection arrangements are configured to releasably connect to a pump or air tube to inflate/deflate the one or more air bladders. In one non-limiting arrangement, the one or more air connection arrangements are a quick-connect arrangement known in the art. The BFRT cuff generally includes one or more valves to maintain and/or release air from the one or more air bladders. In one non-limiting arrangement, the air connection arrangements include one or more valves.

In another and/or alternative non-limiting aspect of the present disclosure, there is provided a motor/pump unit to inflate and/or deflate the one or more air bladders in the BFRT cuff. In one non-limiting embodiment, the housing of the motor/pump unit includes a rechargeable battery. The rechargeable battery can be charged wirelessly and/or via a charge cable. The housing optionally includes a charging port for the rechargeable battery. The motor/pump unit can include an air tube connected at one end to the housing of the motor/pump unit and configured to be connected (e.g., releasably connected, non-releasably connected, etc.) at the other end to an air connection arrangement on the BFRT cuff.

In another and/or alternative non-limiting aspect of the present disclosure, the motor/pump unit includes a sensor to detect the air pressure in the one or more air bladders of the BFRT cuff. The motor/pump unit typically includes electronics (e.g., processor, software code, logic circuits, etc.) that facilitate in the measurement calculations for pressure.

In another and/or alternative non-limiting aspect of the present disclosure, the housing of the motor/pump unit includes a display and one or more user interface buttons. The one or more buttons can include a) a power button, b) one or more arrow or scroll up/down buttons, and/or c) a reset button. The display can be a LED display or the like, and may or may not be a touch screen display. Information on the display can include, but is not limited to, a) battery power level, b) pressure reading, c) warning indicator for too high pressure, d) warning indicator for too low pressure, e) waring indicator of pressure above or below preset % LOP, f) warning indicator for battery level too low, g) information about BFRT, h) arm or leg selection, i) limb occlusion pressure (LOP), j) day and/or time, k) time BFRT cuff pressurized, I) pressure reset, m) system reset, n) deflate BFRT cuff, o) inflate BFRT cuff, p) percent of LOP to which BFRT cuff is to be pressurized, q) preset range of operation for preset % LOP, etc.

In another and/or alternative non-limiting aspect of the present disclosure, the motor/pump unit is configured to 1) pressurize the BFRT cuff until a LOP pressure is reached or a maximum preset pressure (e.g., 400 mmHg, 100-500 mmHg [and all values and ranges therebetween], etc.) is reached, 2) reduce the pressure in the BFRT cuff to a set percent of LOP (% LOP) (e.g., preset % LOP or manually set % LOP), and 3) terminate operation of the air pump after the set % LOP is obtained or after a maximum preset pressure has been obtained.

In another and/or alternative non-limiting aspect of the present disclosure, the BFRT system is configured to maintain the pressure (e.g., Auto Regulate pressure) in the one or more air chambers of the BFRT cuff at a preselected pressure or within a set % LOP during use of the BFRT cuff by a user. During use of the BFRT system, the BFRT system can maintain the pressure in one or more air chambers in the BFRT cuff at a preselected value or a preselected pressure range. For example, a user can preset the pressure in the one or more air chambers in the BFRT cuff at a certain % LOP, at a certain range of % LOP, a certain pressure, and/or at a certain range of pressure. In one non-limiting arrangement, a user can preset or the BFRT system can automatically create a preset for a certain % LOP at which the one or more air chambers in the BFRT cuff are to be inflated during the use of the BFRT cuff by a user, and if one or more pressure sensors detect the pressure in the one or more air chambers exceeding the preset % LOP, the BFRT system causes gas (e.g., air, etc.) to be released from the one or more air chambers until the one or more pressure sensors detect a pressure in the one or more air chambers that indicates that the pressure is now at the preset % LOP, and if one or more pressure sensors detect the pressure in the one or more air chambers below the preset % LOP, the BFRT system causes gas (e.g., air, etc.) to be inserted into the one or more air chambers until the one or more pressure sensors detect a pressure in the one or more air chambers that indicates that the pressure is now at the preset % LOP. In one non-limiting arrangement, a user can preset or the BFRT system can automatically preset create a preset for a certain range of % LOP (e.g., 5-99% of LOP [and all values and ranges therebetween]) or to be within a certain range (±0.001% to ±10% [and all values and ranges therebetween]) of a preselected % LOP that the one or more air chambers in the BFRT cuff are to be inflated during the use of the BFRT cuff by a user, and if one or more pressure sensors detect the pressure in the one or more air chambers exceeding the preset range of LOP% or exceeding the preset range of the preset LOP %, the BFRT system causes gas (e.g., air, etc.) to be released from the one or more air chambers until the one or more pressure sensors detect a pressure in the one or more air chambers that indicates that the pressure is now within the present range of LOP % or within the preset range of the present LOP %, and if one or more pressure sensors detect the pressure in the one or more air chambers below the preset range of LOP% or below the preset range of the preset LOP %, the BFRT system causes gas (e.g., air, etc.) to be inserted into the one or more air chambers until the one or more pressure sensors detect a pressure in the one or more air chambers that indicates that the pressure is now within the present range of LOP % or within the preset range of the present LOP %.

In another and/or alternative non-limiting aspect of the present disclosure, the BFRT system is configured to maintain the pressure (e.g., Auto Regulate pressure) in the one or more air chambers of the BFRT cuff within a range of a preselected pressure or within a set % LOP during use of the BFRT cuff by a user. For example, a user can preset the pressure in the one or more air chambers in the BFRT cuff at a certain % LOP when the BFRT cuff is attached to an arm of the user (e.g., arm - 30% LOP-50% LOP and all values and ranges therebetween) and the BFRT system then maintains the pressure in the one or more air chambers of the BFRT cuff within a certain range encompassing the preset % LOP (e.g., 20% LOP - 60% LOP and all values and ranges therebetween). In one specific example, the BFRT cuff is attached to the user's arm. The BFRT system is set by the user to use a 35% LOP for the BFRT cuff during use of the BFRT cuff by the user. The BFRT cuff then inflates the BFRT cuff to determine the LOP pressure, and then releases the pressure in the BFRT cuff until the pressure in the BFRT cuff is 35% LOP. While the user exercises his/her arm that includes the BFRT cuff, the pressure in the BFRT cuff increases and decreases during arm extension exercise. When the arm is bent, the muscles in the arm cause compression on the BFRT cuff, thereby causing an increase in pressure in the BFRT cuff. If the detected pressure exceeds an upper % LOP setting (e.g., 60 % LOP), the BFRT system causes the pressure in the BFRT cuff to decrease so as to fall below the upper % LOP setting. When the arm is extended, the muscles in the arm decrease compression on BFRT cuff, thereby causing a decrease in pressure in the BFRT cuff. If the detected pressure is below a lower % LOP setting (e.g., 20 % LOP), the BFRT system causes the pressure in the BFRT cuff to increase to cause the pressure to be above the lower % LOP setting. As can be appreciated, the selected % LOP for a particular arm exercise can be manually set by a user or be automatically set by the BFRT system. Also, the upper % LOP and/or lower % LOP for a particular arm exercise can be manually set by a user or be automatically set by the BFRT system. In another example, a user can preset the pressure in the one or more air chambers in the BFRT cuff at a certain % LOP when the BFRT cuff is attached to a leg of the user (e.g., leg - 50% LOP-80% LOP and all values and ranges therebetween) and the BFRT system maintains the pressure in the one or more air chambers of the BFRT cuff within a certain range encompassing the preset % LOP (e.g., 40% LOP - 90% LOP and all values and ranges therebetween). In one specific example, the BFRT cuff is attached to the user's leg. The BFRT system is set by the user to use a 60% LOP for the BFRT cuff during use of the BFRT cuff. The BFRT cuff then inflates the BFRT cuff to determine the LOP pressure, and then releases the pressure in the BFRT cuff until the pressure in the BFRT cuff is 60% LOP. While the user exercises his/her leg that includes the BFRT cuff, the pressure in the BFRT cuff increases and decreases during a leg extension exercise. When the leg is bent, the muscles in the leg cause compression on the BFRT cuff, thereby causing an increase in pressure in the BFRT cuff. If the detected pressure exceeds an upper % LOP setting (e.g., 90 % LOP), the BFRT system causes the pressure in the BFRT cuff to decrease to fall below the upper % LOP setting. When the leg is extended, the muscles in the leg decrease compression on BFRT cuff, thereby causing a decrease in pressure in the BFRT cuff. If the detected pressure is below a lower % LOP setting (e.g., 40 % LOP), the BFRT system will cause the pressure in the BFRT cuff to increase so as to cause the pressure to be above the lower % LOP setting. As can be appreciated, the selected % LOP for a particular leg exercise can be manually set by a user or be automatically set by the BFRT system. Also, the upper % LOP and/or lower % LOP for a particular leg exercise can be manually set by a user or be automatically set by the BFRT system.

In another and/or alternative non-limiting aspect of the present disclosure, the BFRT system is configured have Intermittent Regulation to partially or fully deflate the one or more air chambers of the BFRT cuff when the BFRT system detects that 1) the user is not using the BFRT cuff, or 2) a set exercise is completed and/or a set number of repetitions (reps) is completed. This Intermittent Regulation feature ensures that the BFRT cuff is partially or fully deflated between exercise sets or reps, or when the BFRT cuff is not being used. In one specific example, the BFRT cuff is attached to the user's arm. The user manually selects an exercise routine or enters a custom exercise routine. The BFRT cuff is inflated to determine the LOP pressure, and then releases the pressure in the BFRT cuff until the pressure in the BFRT cuff is reduced to some % LOP. While the user exercises his/her arm that includes the BFRT cuff, the pressure in the BFRT cuff increases and decreases during an arm extension exercise. Such increase and decrease in pressure is used by the BFRT system to 1) determine whether the BFRT cuff is being used, and/or 2) determine a number of user's reps during an exercise routine. If the BFRT system determines that the BFRT cuff is not being used after a certain period of time (e.g., 2-120 seconds and all values and ranges therebetween, 4-10 seconds, etc.), the BFRT system can be design to partially or fully deflate the BFRT cuff until use is again detected and/or a new exercise routine is started by the user or automatically started by the BFRT system. In one non-limiting example, the BFRT system is designed to deflate the BFRT cuff to 0% LOP to 19.9% LOP (and all values and ranges therebetween) when the BFRT cuff is attached to the arm and the BFRT system determines that the BFRT cuff is not being used after a certain period of time. In another non-limiting example, the BFRT system is designed to deflate the BFRT cuff to 0% LOP to 39.9% LOP (and all values and ranges therebetween) when the BFRT cuff is attached to the leg and the BFRT system determines that the BFRT cuff is not being used after a certain period of time. As can be appreciated, the deflation % LOP when the BFRT system determines that the BFRT cuff is not being used after a certain period of time can be manually set by a user or be automatically set by the BFRT system. Also, the time period that the BFRT system uses to determine non-use b can be manually set by a user or be automatically set by the BFRT system. In another non-limiting example, when the BFRT system is designed to partially or fully deflate the BFRT cuff after the BFRT system has determined that 1) the number of reps selected by the user has been completed, and/or 2) a selected exercise routine has been completed by the user, the time period before the BFRT system allows the user to again have the BFRT cuff inflated for another exercise routine can be a manually selected time period or a preprogrammed time period. Generally, such time period is 5 seconds to 4 minutes (and all values and ranges therebetween), and typically 30 seconds to 3 minutes, and more typically 1-2 minutes.

In another and/or alternative non-limiting aspect of the present disclosure, the BFRT system can include preprogrammed exercise routines for the arm and/or leg.

In another and/or alternative non-limiting aspect of the present disclosure, the BFRT system can allow the user to customize and/or manually input exercise routines for the arm and/or leg.

In another and/or alternative non-limiting aspect of the present disclosure, the BFRT cuff can have a urethane-coated air bladder. The urethane coating on the bladder adds to user comfort by reducing pinching of the skin by the bladder when inflated. The urethane coating can also optionally fill in the folds created by the bladder when inflated.

In another and/or alternative non-limiting aspect of the present disclosure, one non-limiting method of operation of the motor/pump unit and BFRT cuff is as follows: a) removably placing a BFRT cuff (e.g., using a hook and loop strap fastener, etc.) onto a user's limb (e.g., arm, leg, etc.), b) attach the pump or pump air hose/tube to the BFRT cuff if not already connected, c) turning on motor/pump unit if not already on, d) optionally allow or cause the motor/pump unit to calibrate to ambient pressure, e) optionally select limb (arm, leg, etc.) on the motor/pump unit (e.g., select limb listed on display of housing of the motor/pump unit, etc.), f) select % LOP (e.g., using display and one or more selection keys on housing of the motor/pump unit, the user selects a desired % LOP or a default % LOP can be selected by the user), which % LOP is generally 20%-80% and all values therebetween of the LOP, g) cause air pump to be activated to pressurize BFRT cuff until the LOP on the limb is detected, h) cause air pump and/or motor/pump unit to reduce pressure in the BFRT cuff until the selected % LOP is detected in the BFRT cuff, i) terminate operation of the air pump after the % LOP in the BFRT cuff is obtained, and j) optionally disconnect the air pump or the air hose/tube from the BFRT cuff. The % LOP is obtained in the BFRT cuff by the motor/pump unit first pressurizing the BFRT cuff until the LOP of the limb is detected and thereafter removing or allowing air to be removed from the BFRT cuff until the % LOP is obtained. The air pump typically immediately stops inflating the BFRT cuff once LOP in the limb is detected and thereafter causes the deflation of the BFRT cuff to the selected % LOP. For example, if LOP is detected at a pressure of 200 mmHg, then 50% LOP would be 100 mmHg and 75% LOP would be 150 mmHg. After the user completes use of the BFRT cuff when inflated to the selected % LOP, the user can a) manually partially or fully deflate the BFRT cuff and thereafter remove the BFRT cuff from the limb, or b) have the motor/pump unit partially or fully deflate the BFRT cuff and thereafter remove the BFRT cuff from the limb. If the motor/pump unit is not connected to the BFRT cuff, the motor/pump unit is first connected to the BFRT cuff prior to having the motor/pump unit partially or fully deflate the BFRT cuff.

One non-limiting object of the present disclosure is the provision of a method for exercising using a blood flow restriction training (BFRT) cuff comprising: (a) providing a BFRT cuff, said BFRT cuff formed of a flexible outer material, an air chamber at least partially encapsulated by said flexible outer material, an air input port in fluid communication with said air chamber, and a connection strap configured to secure said BFRT cuff to a limb of a user, at least a portion of said air input port positioned on an exterior surface of said BFRT cuff, said air input port including an air valve, said flexible outer material having a length of at least 20 cm and a width of least 5 cm; (b) providing a motor/pump unit, the motor/pump unit configured to at least partially inflate said BFRT cuff, the motor/pump unit including a housing and an air connection arrangement that fluidly connects said housing to said BFRT cuff via said air input port to enable pumped air from the motor/pump unit to flow to and at least partially inflate said BFRT cuff, said housing having a size less than 6.55 × 10³ cm³ (400 cubic inches) and a weight less than 1.13 kg (40 ounces), said housing at least partially contains i) pump (e.g., an electric air pump, etc.) , ii) a power source, and iii) a pressure sensor; (c) placing said BFRT cuff on a user's limb; (d) connecting said BFRT cuff to the motor/pump unit to enable air flow between said BFRT cuff the motor/pump unit; (e) selecting a pressure value or percent of LOP, said pressure value less than said LOP, said percent no more than 90%; (f) causing the motor/pump unit to supply air to said BFRT cuff thereby causing said BFRT cuff to inflate until said LOP of said limb is obtained; (g) terminating air flow from the motor/pump unit to said BFRT cuff after said LOP has been obtained for the limb; (h) causing the motor/pump unit to begin reducing pressure in said BFRT cuff within 10 seconds after said LOP is obtained in said BFRT cuff and continue reducing pressure in said BFRT cuff until said pressure value that was selected or said percent of said LOP that was selected is obtained in said BFRT cuff; (i) optionally disconnecting said BFRT cuff from the motor/pump unit so that the motor/pump unit cannot supply air to said BFRT cuff; and, (j) exercising the user's limb while said BFRT cuff is at the selected pressure or the selected percent of said LOP.

Another and/or alternative non-limiting object of the present disclosure is the provision of a method for exercising using a BFRT cuff that further includes the steps of: (k) reconnecting said BFRT cuff to the motor/pump unit to enable air flow between said BFRT cuff the motor/pump unit after the user has completed use of said BFRT cuff on the limb at said pressure value that was selected or said percent of said LOP that was selected; and, (I) causing the motor/pump unit to remove air from said BFRT cuff to reduce pressure in said BFRT cuff below said pressure value that was selected or said percent of said LOP that was selected.

Another and/or alternative non-limiting object of the present disclosure is the provision of a method for exercising using a blood flow restriction training (BFRT) cuff that further includes the step of removing said BFRT cuff from said limb after said step of exercising the user's limb.

Another and/or alternative non-limiting object of the present disclosure is the provision of a method for exercising using a blood flow restriction training (BFRT) cuff wherein said housing of the motor/pump unit includes a circuit board, a processor, a display, a power button, and one or more selection or scroll buttons, said one or more selection or scroll buttons configured to enable manual operation and/or control of the motor/pump unit, said display displaying three or more types of information selected from the group consisting of a) measured pressure, b) power or charge level of said power source, c) a warning symbol when some type of warning event has occurred, d) selected % LOP, e) selected pressure, f) instructional information as to how to use the motor/pump unit and/or BFRT cuff, and g) arm or leg selection options for BFRT cuff.

Another and/or alternative non-limiting object of the present disclosure is the provision of a method for exercising using a BFRT cuff further including the step of turning on or powering up the motor/pump unit, the motor/pump unit configured to calibrate with ambient pressure after the motor/pump unit is turned on or powered up.

Another and/or alternative non-limiting object of the present disclosure is the provision of a method for exercising using a BFRT cuff that further includes the step of selecting the limb upon which said BFRT cuff is to be place.

Another and/or alternative non-limiting object of the present disclosure is the provision of a method for exercising using a BFRT cuff that includes the steps of a) providing a BFRT cuff, said BFRT cuff including i) a flexible outer material, ii) an air chamber at least partially encapsulated by said flexible outer material, and iii) a connection arrangement configured to secure said BFRT cuff to a limb of a user, said flexible outer material having a length of at least 20 cm and a width of least 5 cm; b) providing a motor/pump unit, the motor/pump unit configured to at least partially inflate said BFRT cuff, the motor/pump unit including a housing and an air connection arrangement that fluidly connects said housing to said BFRT cuff to enable pumped air from the motor/pump unit to flow to and at least partially inflate said BFRT cuff, said housing a size less than 6.55 × 10³ cm³ (400 cubic inches) and a weight less than 1.81 kg (4 lbs), said housing at least partially containing i) an air pump, ii) a power source, and iii) a pressure sensor; c) placing said BFRT cuff on a user's limb; d) connecting said BFRT cuff to the motor/pump unit to enable air flow between said BFRT cuff and the motor/pump unit; e) selecting a pressure value or percent of limb occlusion pressure (LOP), said pressure value less than said LOP, said percent no more than 90%; f) causing the motor/pump unit to supply air to said BFRT cuff to thereby cause said BFRT cuff to inflate until said LOP of said limb is obtained; g) terminating air flow from the motor/pump unit to said BFRT cuff after said LOP has been obtained for the limb; h) reducing pressure in said BFRT cuff within 0.001-30 seconds after said LOP is obtained in said BFRT cuff until said pressure value is said selected pressure value or said percent of said LOP; and, i) exercising the user's limb while said BFRT cuff is at said selected pressure value or said selected percent of said LOP; and wherein the motor/pump unit automatically activates said air pump when said pressure in said air chamber falls a certain amount below said selected pressure value or said percent of said LOP during said step of exercising to increase said pressure in said air chamber to said selected pressure value or said percent of said LOP.

Another and/or alternative non-limiting object of the present disclosure is the provision of a method for exercising using a BFRT cuff that includes the steps of a) providing a BFRT cuff, said BFRT cuff formed of a flexible outer material, an air chamber at least partially encapsulated by said flexible outer material, an air input port in fluid communication with said air chamber, and a connection strap configured to secure said BFRT cuff to a user's limb, at least a portion of said air input port positioned on an exterior surface of said BFRT cuff, said air input port including an air valve, said flexible outer material having a length of at least 20 cm and a width of least 5 cm; b) providing a motor/pump unit, said motor/pump unit configured to at least partially inflate said BFRT cuff, the motor/pump unit including a housing and an air connection arrangement that fluidly connects said housing to said BFRT cuff via said air input port to enable pumped air from the motor/pump unit to flow to and at least partially inflate said BFRT cuff, said housing having a size less than 6.55 × 10³ cm³ (400 cubic inches) and a weight less than 1.81 kg (4 lbs), said housing at least partially containing i) an electric air pump, ii) a power source, and iii) a pressure sensor; c) placing said BFRT cuff on a user's limb; d) connecting said BFRT cuff to the motor/pump unit to enable air flow between said BFRT cuff and the motor/pump unit; e) selecting a pressure value or percent of limb occlusion pressure (LOP), said pressure value less than said LOP, said percent no more than 95% of LOP (e.g., 5-95% and all values and ranges therebetween], 50-90%, etc.); f) causing the motor/pump unit to supply air to said BFRT cuff to thereby cause said BFRT cuff to inflate until said LOP of said limb is obtained; g) terminating air flow from the motor/pump unit to said BFRT cuff after said LOP has been obtained for the limb; h) causing the motor/pump unit to begin reducing pressure in said BFRT cuff within some preset time (e.g., 0.001-2 minutes [and al values and ranges therebetween], 0.001-10 seconds, etc.] after said LOP is obtained in said BFRT cuff and continue to reduce pressure in said BFRT cuff until said pressure value that was selected or said percent of said LOP that was selected is obtained in said BFRT cuff; i) optionally disconnecting said BFRT cuff from the motor/pump unit so that the motor/pump unit cannot supply air to said BFRT cuff; j) optionally maintaining the motor/pump unit on the BFRT cuff, and optionally enabling the motor/pump unit to maintain/control a pressure of the BFRT cuff (e.g., Auto Regulation of pressure) during use; k) exercising the user's limb while said BFRT cuff is at the selected pressure or the selected percent of said LOP, and l) optionally providing Intermittent Regulation of pressure of the BFRT cuff.

Other objects, advantages, and novel features of the present disclosure will become apparent from the following description of the disclosure when considered in conjunction with the accompanying drawings.

### BRIEF DESCRIPTION OF DRAWINGS

FIGS. 1 and 1A illustrate two BFRT cuffs and a motor/pump unit for each of the BFRT cuffs in accordance with the present disclosure.
FIG. 2 illustrates a top view of a non-limiting display on the housing of the motor/pump unit of FIG. 1.
FIG. 3 is a front view of a non-limiting housing of the motor/pump unit of FIG. 1.
FIGS. 4-12 are illustrations of flow diagrams for the operation of the motor/pump unit and BFRT cuff in accordance with one non-limiting aspect of the present disclosure.
FIG. 13 illustrates a side view of another BFRT cuff system in accordance with the present disclosure that includes a motor/pump unit connected to a portion of the BFRT cuff.
FIG. 14 illustrates a front perspective view of the BFRT cuff system of FIG. 13.
FIG. 15 illustrates an enlarged front perspective view of the BFRT cuff system of FIG. 13.
FIG. 16 illustrates a rear perspective view of the BFRT cuff system of FIG. 13 wherein the motor/pump unit is disconnected from the BFRT cuff.
FIG. 17 illustrates a top perspective view of the BFRT cuff system of FIG. 16.

### DETAILED DESCRIPTION

A more complete understanding of the articles/devices, processes and components disclosed herein can be obtained by reference to the accompanying drawings. These figures are merely schematic representations based on convenience and the ease of demonstrating the present disclosure, and are, therefore, not intended to indicate relative size and dimensions of the devices or components thereof and/or to define or limit the scope of the exemplary embodiments.

Although specific terms are used in the following description for the sake of clarity, these terms are intended to refer only to the particular structure of the embodiments selected for illustration in the drawings and are not intended to define or limit the scope of the disclosure. In the drawings and the following description below, it is to be understood that like numeric designations refer to components of like function.

The singular forms "a," "an," and "the" include plural referents unless the context clearly dictates otherwise.

As used in the specification and in the claims, the term "comprising" may include the embodiments "consisting of" and "consisting essentially of." The terms "comprise(s)," "include(s)," "having," "has," "can," "contain(s)," and variants thereof, as used herein, are intended to be open-ended transitional phrases, terms, or words that require the presence of the named ingredients/steps and permit the presence of other ingredients/steps. However, such description should be construed as also describing compositions or processes as "consisting of" and "consisting essentially of" the enumerated ingredients/steps, which allows the presence of only the named ingredients/steps, along with any unavoidable impurities that might result therefrom, and excludes other ingredients/steps.

Numerical values in the specification and claims of this application should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 5.08 cm to 25.4 cm (from 2 inches to 10 inches)" is inclusive of the endpoints, 5.08 cm (2 inches) and 25.4 cm (10 inches), and all the intermediate values).

The terms "about" and "approximately" can be used to include any numerical value that can vary without changing the basic function of that value. When used with a range, "about" and "approximately" also disclose the range defined by the absolute values of the two endpoints, e.g. "about 2 to about 4" also discloses the range "from 2 to 4." Generally, the terms "about" and "approximately" may refer to plus or minus 10% of the indicated number.

In accordance with the present disclosure, there is provided a BFRT system **20** that includes a BFRT cuff **100** and motor/pump unit **200.**

BFRT **100** cuff includes an inflatable air system formed of one or more two layers of material. In one non-limiting embodiment, the inflatable air system includes two layers of material coupled together to create one or more air chambers to be inflated with air and/or some other gas. In one non-limiting configuration, BFRT cuff **100** is a single air chamber inflatable cuff. In another non-limiting configuration, BFRT cuff **100** includes two or more air chambers. The configuration and shape of the one or more air chambers is non-limiting. The inflatable air system is configured in BFRT cuff **100** such that inflation of the inflatable air system produces compression on a target compression zone that in turn produces a restriction of blood flow in the venous system of a user. BFRT cuff **100** includes an outer material cover **102** having a cavity (not shown) for containing the inflatable air system to partially or fully isolate the inflatable air system from the skin of the user and/or to provide protection to the inflatable air system, and/or to provide structural strength to BFRT cuff **100.** The inflatable air system can optionally be secured in the cavity to one or more inner surface of outer material cover **102** (e.g., stitching, adhesive, melted connection, etc.). Oter material cover **102** is generally made of a flexible and durable material (e.g., nylon, Kevlar^{™}, etc.). In one non-limiting embodiment, outer material cover **102** is formed of an inelastic or non-stretch material. Outer material cover **102** is configured to a) facilitate in distributing the force applied by the inflatable air system to the limb of a user, b) reduce pinching of the user's skin during the inflation of the inflatable air system, and/or c) provide friction between BFRT cuff **100** and the user's skin to inhibit or prevent rotation of BFRT cuff **100** on the user during use.

BFRT cuff **100** generally includes a connection arrangement to facilitate in the securing of BFRT cuff **100** to a user's limb. As illustrated in 1, BFRT cuff **100** includes a loop/loop like material **110** on the outer surface that is configured to releasably connect to a hook/loop material on a strap **120.**

BFRT cuff **100** includes one or more input ports **130** (e.g., air input port, data/communication input port, power input port, etc.). The one or more input ports can be in fluid communication with the inflatable air system to allow a fluid (e.g., air) to flow into and out of the inflatable air system. Input port **130** generally includes a valve component (not shown). The location of the one or more input ports **130** on the BFRT is non-limiting. Generally, at least one input port **130** is located on an outwardly facing surface of the outer material cover **102.** One or more of the input ports **130** is configured to be connected to a motor/pump unit **200** (e.g., portable motor/pump, non-portable motor/pump, permanently connected motor/pump, detachably connected motor/pump, etc.). The one or more input ports **130** can optionally include a one-way valve (not shown) to prevent undesired deflation of the air inflatable system. The one or more input ports **130** can optionally be configured to allow for manual deflation of the air inflatable system.

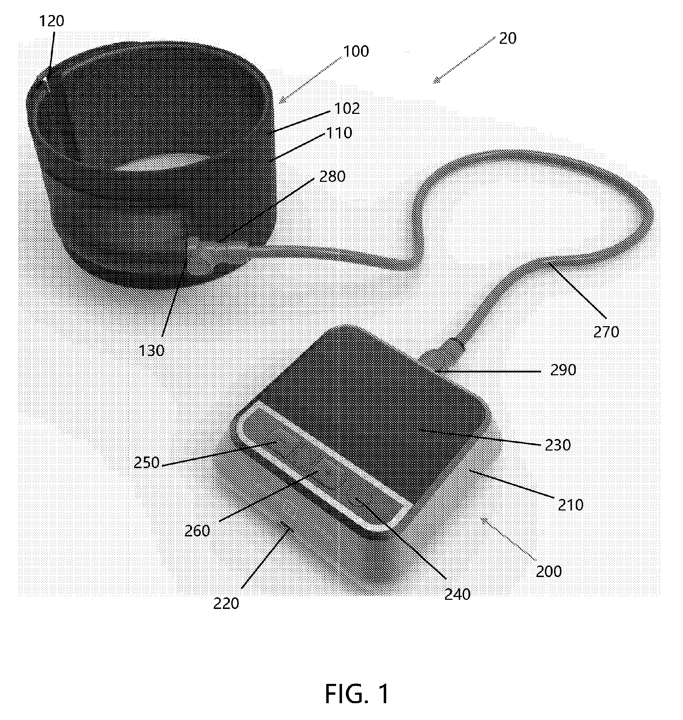

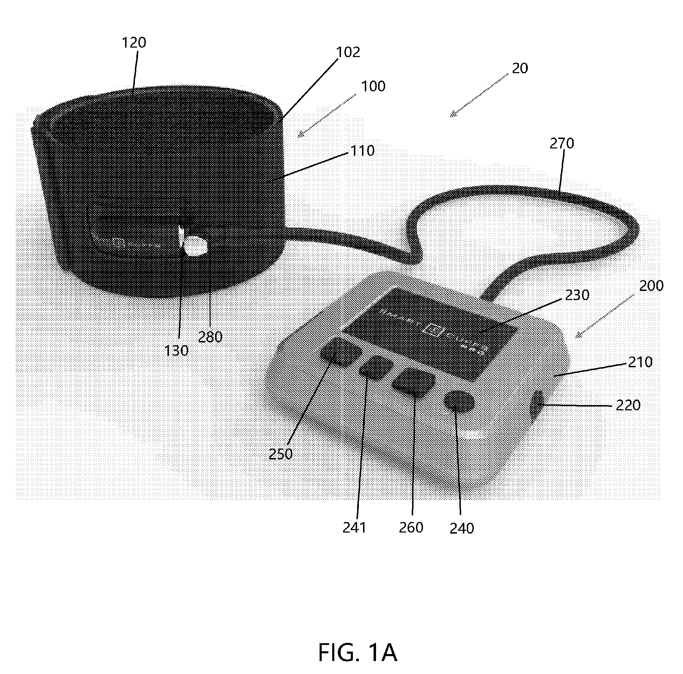

As illustrated in FIGS. 1, 1A, 2 and 3, the BFRT cuff **100** has a motor/pump unit **200** connected to an outer surface **112** of outer material cover **110.** In particular, the outer material cover **110** includes a first end **114** and second end **116** and the motor/pump unit **200** is connected to a portion of the outer material cover located closest to first end **114.** As can be appreciated, the motor/pump unit **120** can be located on any portion of the outer material cover **110.** Although not shown, the outer material cover **110** is connected to an input port so the motor/pump unit **120** can inflate/deflate the BFRT cuff **100.**

Motor/pump unit **200** includes a housing **210** that at least partially contains a) a motor (not shown); b) a pump (not shown); c) optionally a power source (not shown); d) one or more pressure sensors (not shown); e) circuit board and other electronics (not shown); f) one or more processors (e.g., controller, etc.); g) one or more pressure gauge(s) (not shown); h) software (not shown); i) one or more display(s) **230** (e.g., LED screen, OLED screen, etc.); j) optionally a power port (not shown); k) optionally a data port **220** (e.g., micro USB port, etc.); I) optional GPS system (not shown); m) optional wireless electronics (e.g., transmitter and/or receiver, etc.) to send/receive wireless signals (not shown); n) one or more buttons **240, 241** (e.g., power button, home button, power off button, etc.); o) one or more selection or scroll buttons **250, 260** to enable manual operation/control of motor/pump unit **200;** p) one or more switch(es) (not shown) to enable manual operation/control of motor/pump unit **220**; q) connection arrangement **280** to permanently or releasably connect motor/pump unit **220** to the outer material cover **110;** r) air hose port **290;** and/or s) optional lights and/or other indicators (not shown) to indicate a mode and/or operation and/or status of motor/pump unit **200**. The shape and size of housing **210** is non-limiting. The material used to form housing **210** is generally a durable material (e.g., plastic, metal, composite, etc.). Motor/pump unit **200** is generally a portable motor/pump unit **200** that can be easily handled by a user. Generally, the volume of housing **210** of motor/pump unit **200** is no more than 8.19 × 10³ cm³ (500 cubic inches), typically 8.19-8.19 × 10³ cm³ (0.5-500 cubic inches) (and all values and ranges therebetween), and more typically 16.39-819.35 cm³ (1-50 cubic inches). Generally, the weight of motor/pump unit 200, including housing 210 and air hose 270, is no more than 4.54 kg (10 lbs), typically 0.0227-4.54 kg (0.05-10 lbs) (and all values and ranges therebetween), and more typically 0.0907-1.36 kg (0.2-3 lbs).

As illustrated in FIG. 1, a first end of an air hose **270** is connected to the air hose port **290** on housing **210.** Air hose **270** can be permanently or detachably connected to the housing **210.** The second end of air hose **280** includes a hose connector **280** configured to releasably connect to air input port **130** on BFRT cuff **100.** Generally, the type of connection between hose connector **280** and air input port **130** is a quick-connect connection arrangement; however, this is not required.

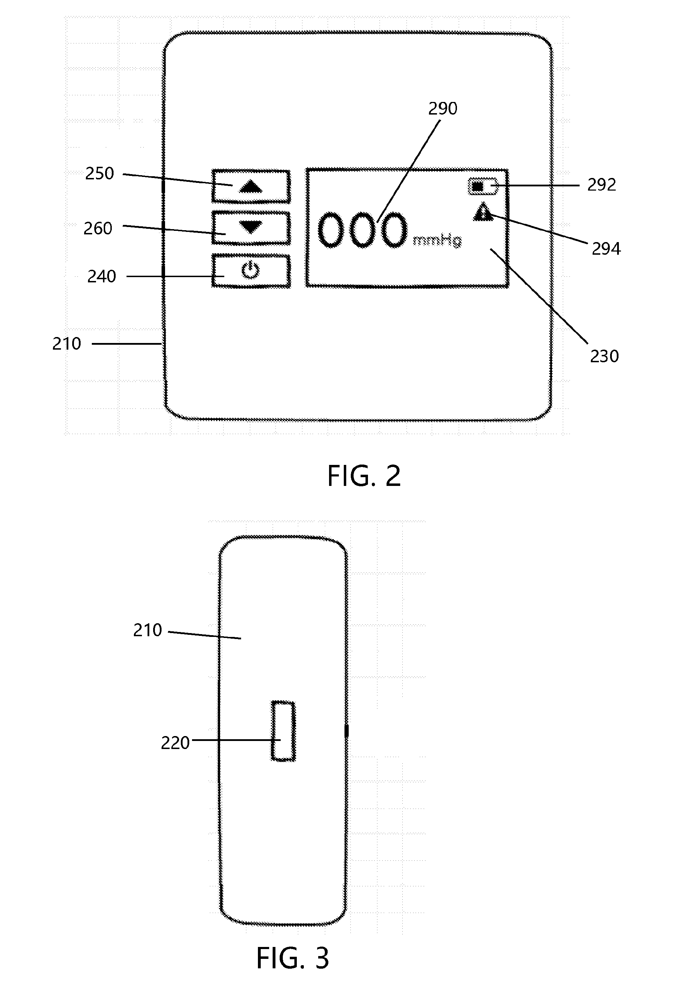

As illustrated in FIGS. 1 and 3, the front face of housing **210** includes a power port/data port **220.** The type of connection is non-limiting. As can be appreciated, the power port/data port **220** can be located on other regions of housing **220**. The power port/data port **220** can optionally recharge the optional battery in the housing **210** and/or power the motor/pump unit **200**. The power port/data port **220** can optionally 1) provide software updates to the motor/pump unit **200** and/or allow for repair/reset of the motor/pump unit **200,** 2) obtain use/diagnostic information from the motor/pump unit **200,** and/or 3) download/upload user information and/or BFRT use information.

As illustrated in FIGS. 1 and 2, two types of button and display configurations are illustrated. As can be appreciated, other types of button and display configurations can be used on housing **210** of motor/pump unit **200**. As illustrated in FIG. 1, the top of housing **210** includes a large display **230** covering about 50-80% (and all values and ranges therebetween) of the top surface of the housing **210.** The display **230** can be a LED display or the like. Positioned below the display **230** is a power button **220** and two arrow or scroll buttons **250, 260.** The power button turns the motor/pump unit **200** on and off. The power button **220** can also optionally reset the motor/pump unit **200**. The two arrow or scroll buttons **250, 260** can be used to select items displayed on the display **230** (e.g., select % LOP, select if BFRT cuff is to be used on leg or arm, select time of exercise, select if % LOP used on one limb is to be also used on another limb, deflate BFRT cuff, inflate BFRT cuff, reset motor/pump unit **200,** select day, select time, remove/reset warning on display, perform diagnostics on motor/pump unit **200,** update on motor/pump unit **200** software/firmware, cause BFRT cuff to be pressurized to LOP for a user limb and then to depressurize BFRT cuff to selected or programmed % LOP, activate/deactivate wireless mode, wirelessly pair motor/pump unit **200** with BFRT cuff **100,** select mode of operation of motor/pump unit **200,** unlock/lock motor/pump unit **200** to BFRT cuff **100,** set inflation pressure, set automatic pressure maintaining mode, etc.). As can be appreciated, the arrow or scroll buttons **250, 260** can be used for other or additional functions.

As illustrated in FIG. 3, the top of the housing **210** includes a display **230,** a power button **240,** and two arrow or scroll buttons **250, 260;** however, the orientation of the display **230** and two arrow or scroll buttons **250, 260** on the top of the housing is different. The size of the display in FIG. 3 is smaller than in FIG. 1. As illustrated in FIG. 3, the size of the display covers about 12-40% (and all values and ranges therebetween) of the top surface of the housing **210.** The power button **240** and the two arrow or scroll buttons **250, 260** are positioned to the left side of the display **230** as opposed to the bottom of the display **230** as illustrated in FIG. 1. The function of the power button **240** and two arrow or scroll buttons **250, 260** illustrated in FIG. 3 can be the same or different from the function of power button **240** and the two arrow or scroll buttons **250, 260** described above with reference to FIG. 1.

As illustrated in FIG. 3, the display shows the measured pressure in mmHg, the power or charge level of the battery in housing **210,** and optionally displays a warning symbol if some type of warning event has occurred (e.g., battery power level too low, malfunction of air pump, malfunction of electronic/processor of motor/pump unit **200,** maximum measured/detected pressure exceeded, etc.). As can be appreciated, displays **230** of FIGS. 1 and 3 can display other or additional material (e.g., selected % LOP, operational information for motor/pump unit **200** and/or BFRT cuff **100,** LOP pressure, pressure at selected % LOP, operational state or step of the motor/pump unit **200** [e.g., inflating BFRT cuff, obtaining limb LOP, deflating BFRT cuff to selected % LOP, fully deflating BFRT cuff, etc.], manual selection of % LOP, selection of arms or legs for BFRT cuff inflation, select to fully deflate BFRT cuff, time remaining to fully charge the battery, whether the air pump is on/off, etc.).

### Operation of BFRT System

The operation of BFRT system **20** illustrated in FIGS. 1-3 will now be described:
Step 1 - Turning on or powering up motor/pump unit **200** (if not already turned on). The turning on or powering up of th motor/pump unit **200** can be accomplished by pressing power button **240** on housing **210.** If motor/pump unit **200** has been turned off or powered down, motor/pump unit **200** can optionally be configured to calibrate with the ambient pressure when motor/pump unit **200** is tuned on or powered up. Generally, motor/pump unit **200** turning on or powering up of motor/pump unit **200** will cause the display **230** to be turned on.
Step 2 - Optionally charge battery in housing **210** of motor/pump unit **200** (if not already sufficiently charged). Generally, the battery in housing **210** should be charged at least 30-50% (and all values and ranges therebetween) of a full charge prior to operation of motor/pump unit **200** to inflate BFRT cuff **100.** The battery charge level **292** can be optionally displayed on display **230** of housing **210.** In one non-limiting embodiment, the battery in housing **210** should be charged at least 50-100% (and all values and ranges therebetween) of a full charge prior to operation of motor/pump unit **200** to inflate BFRT cuff **100.** Motor/pump unit **200** can optionally be configured to cause a warning **294** to be displayed on display **230** of housing **210** when 1) the battery charge level is too low, and/or 2) occlusion has been detected when occlusion was not being tested thereby causing an auto-vent protocol to engage. As can be appreciated, motor/pump unit **200** can be configured such that other or alternative events can cause warning **294** to be displayed on display **230**. Motor/pump unit **200** can optionally be configured to prevent operation of the air pump when the battery charge level is too low.
Step 3 - Optionally select the limb (e.g., arm or leg) upon which BFRT cuff **100** is to be placed. Motor/pump unit **200** can be optionally programmed such that display **230** on housing **210** requests the user to select using one or both of two arrow or scroll buttons **250, 260** or by touching the display (if the display is a touch screen display) on the limb upon which that BFRT cuff **100** is to be attached during the inflation of BFRT cuff **100.** This step and feature of portable motor/pump unit **200** is optional.
Step 4 - Place BFRT cuff **100** on the desired limb of the user. The step of placing BFRT cuff **100** on the desired limb of the user can occur at any time prior to motor/pump unit **200** causing BFRT cuff **100** to be inflated. During the step of placing BFRT cuff **100** on the desired limb of the user, proper size BFRT **100** cuff is selected (generally there are different BFRT cuff sizes for the arms and legs), and then BFRT cuff **100** is fitted about the user's limb, and connection strap (e.g., hook and loop fastener strap, etc.) removably secures BFRT cuff **100** to the limb.
Step 5 - Select % LOP. Motor/pump unit **200** can be is configured to allow the user to select the desired % LOP to be used during the exercise of the limb. The % LOP can be selected by the user using one or both of two arrow or scroll buttons **250, 260** or by touching the display (if the display is a touch screen display). Motor/pump unit **200** can optionally be configured to not permit a selected % LOP greater than 90%. In one non-limiting embodiment, motor/pump unit **200** allows the user to only select a % LOP from 20-85% and all values and ranges therebetween. Motor/pump unit **200** can optionally provide a suggested % LOP based on the input by the user of the limb upon which BFRT cuff **100** is to be connected. Motor/pump unit **200** can optionally have preprogrammed maximum and/or minimum % LOP values so that a user is optionally unable to selected a % LOP that is greater than the maximum preprogrammed % LOP value and/or the user is optionally unable to selected a % LOP that is less than the minimum preprogrammed % LOP value.
Step 6 - Connect motor/pump unit **200** to BFRT cuff **100** prior to activating the pump of the motor/pump unit **200**. The step of connecting motor/pump unit **200** to BFRT cuff **100** can occur at any time prior to motor/pump unit **200** causing BFRT cuff **100** to be inflated. As illustrated in FIG. 1, motor/pump unit **200** can be attached to BFRT cuff **100** by the connection of the air tube **270** to air input port **130** of BFRT cuff **100.** As can be appreciated, motor/pump unit **200** can be connected to BFRT cuff **100** by other or additional arrangements.
Step 7 - Cause air pump of motor/pump unit **200** to activate to cause BFRT cuff **100** to be inflated to the LOP of the limb upon which BFRT cuff **100** is attached. Motor/pump unit **200** can be caused to be activated by the user using one or both of the two arrow or scroll buttons **250, 260** or by touching the display (if the display is a touch screen display), or automatically after the % LOP or selected pressure is selected by the user. Motor/pump unit **200** can optionally be configured to allow the air pump to continue to pump air into BFRT cuff **100** until a maximum preset air pressure is reached in BFRT cuff **100.** Motor/pump unit **200** can optionally be configured to stop the operation of the air pump once the maximum preset air pressure is reached in BFRT cuff **100,** and thereafter optionally cause BFRT cuff **100** to be partially or fully deflated. Such a feature can be used to reduce or prevent damage to BFRT cuff **100** and/or reduce or prevent harm to the user. Motor/pump unit **200** is configured to terminate the flow of air to BFRT cuff **100** once the LOP in the limb has been detected. The LOP can be measured by use of the oscillometric method by inflating up the BFRT cuff **100** until it senses full occlusion (no arterial pulse) then taking an array average/mean pressure. Motor/pump unit **200** can be configured to sense full occlusion as fast as a half a millisecond such that motor/pump unit **200** can take an average pressure in the span of full occlusion. Thereafter, motor/pump unit **200** is configured to allow or cause air to be removed BFRT cuff **100** until the selected % LOP is reached in BFRT cuff **100.** Generally, motor/pump unit **200** is configured to allow or cause air to be removed from BFRT cuff **100** in no more than 10 seconds (e.g., 0.001-10 seconds and all values and ranges therebetween) of motor/pump unit **200** determining or sensing that the LOP has been obtained, and typically motor/pump unit **200** is configured to allow or cause air to be removed from BFRT cuff **100** in no more than 5 seconds of motor/pump unit **200** determining or sensing that the LOP has been obtained, and more typically motor/pump unit **200** is configured to allow or cause air to be removed from BFRT cuff **100** in more than 2 seconds of motor/pump unit **200** determining or sensing that the LOP has been obtained.

The removal of air from BFRT cuff **100** can be by use of a valve in the motor/pump unit **200** that is activated to allow release of air that is flowing from BFRT cuff **100** through air tube **270** and to motor/pump unit **200,** and/or the air pump in motor/pump unit **200** can be configured to draw air from BFRT cuff **100** via air tube **270**.

Once the % LOP or selected pressure has been obtained in BFRT cuff **100,** the air pump terminates operation (if operating) and/or the air release valve in motor/pump unit **200** closes so that no further air is allowed to escape from BFRT cuff **100** after % LOP or selected pressure has been obtained in BFRT cuff **100.** Motor/pump unit **200** can optionally be configured to provide a visual signal via display **230** on housing **210** and/or provide an audible sound once the user selected % LOP or selected pressure in BFRT cuff **100** has been obtained.

Step 8 - Optionally detach motor/pump unit **200** from BFRT cuff **100** after the % LOP has been obtained in BFRT cuff **100.** The detachment of motor/pump unit **200** from BFRT cuff **100** can be accomplished by disconnecting hose connector **280** of air hose **270** from air input port **130** of BFRT cuff **100.** As can be appreciated, motor/pump unit **200** can be disconnected from BFRT cuff **100** in other manners. The disconnecting of motor/pump unit **200** from BFRT cuff **100** results in motor/pump unit **200** not regulating the pressure in BFRT **100** cuff while the user exercises a limb while BFRT cuff **100** is connected and partially inflated on the limb.

Step 9 - Optionally Auto Regulate pressure in BFRT cuff **100** during use of BFRT cuff **100** by the user. If Auto Regulation is used, motor/pump unit **200** is connected to BFRT cuff **100** during use of BFRT cuff **100** by the user. The Auto Regulation of the pressure of BFRT cuff **100** is used to ensure that proper inflation pressure of BFRT cuff **100** is maintained during the use of BFRT cuff **100.**

Step 10 - Deflate BFRT cuff **100.** The partial or full deflation of BFRT cuff **100** can be accomplished by 1) opening an air release valve on BFRT cuff **100,** 2) reconnecting motor/pump unit **200** to BFRT cuff **100** (if not already connected - reconnecting is not required when motor/pump unit **200** remains on BFRT cuff **100** during the use of BFRT cuff **100)** and then causing motor/pump unit **200** to cause air to be removed from BFRT cuff **100** (e.g., activating the air pump to cause the air pump to draw air from BFRT cuff **100,** and through air tube **270** and then out from housing **210,** causing a valve in motor/pump unit **200** to open to allow air to flow from BFRT cuff **100,** through air tube **270** and out through the open valve, etc.). The deflation Step includes the optional Intermittent Regulation feature of the BFRT system.

Step 11 - Remove BFRT cuff **100** from the user's limb. After the user has completed the exercises for the limb using BFRT cuff **100,** the user can remove BFRT cuff **100** from the limb after BFRT cuff **100** has been partially or fully deflated.

The above steps can be modified if the user wishes to perform multiple exercises of the limb using different % LOP. For example, if a user after an exercise wants to continue exercise of the limb but using a different LOP, then after Step 9, the user repeats one or more or Steps 1-3 and 5-10.

Motor/pump unit **200** can optionally be configured to allow a user to select the same % LOP or pressure used on one limb for use on another limb. For example, if a user uses BFRT cuff **100** on his right arm and selected a % LOP of 50% (which was a pressure of 190 mmHg), motor/pump unit **200** can optionally be configured to allow the user to use same BFRT cuff pressure when BFRT cuff **100** is connected to the left arm without having motor/pump unit **200** having to determine the LOP of the left arm. In such an arrangement, motor/pump unit **200** will merely cause BFRT cuff **100** on the left arm to inflate to the same pressure as was used on the right arm. When such a configuration is available on motor/pump unit **200,** such option will typically be displayed on display **230** of housing **210.**

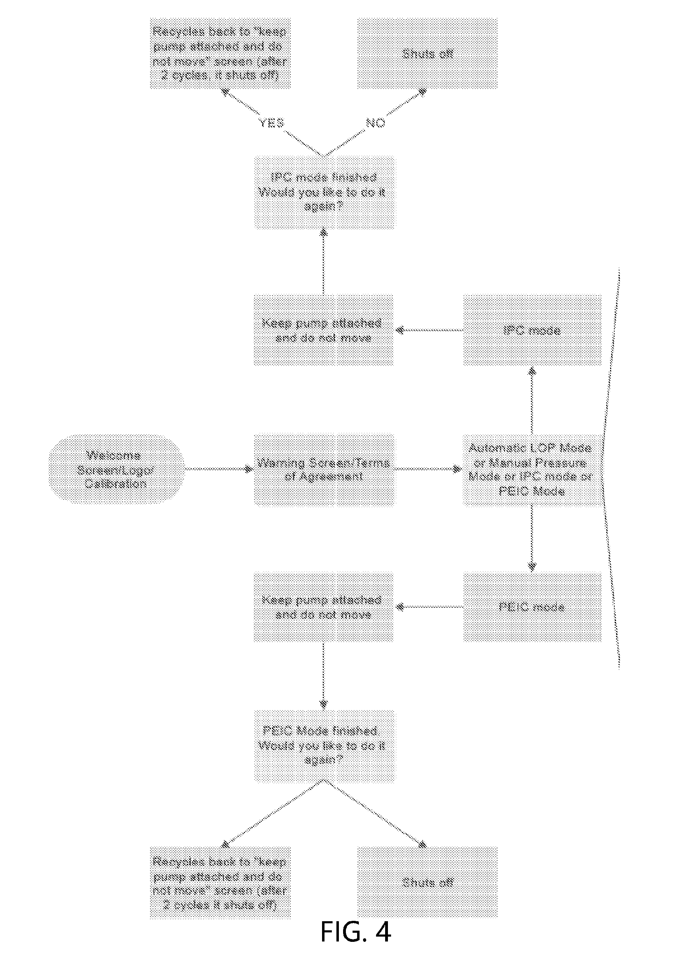

Referring now to FIGS. 4-12, there are illustrations of flow diagrams for the operation of the motor/pump unit **200** and BFRT cuff **100** in accordance with one non-limiting aspect of the present disclosure. FIG. 4 illustrates that the motor/pump unit **200** can operate in either the Automatic LOP mode or the Manual Pressure Mode. As it can be appreciated, the motor/pump unit **200** can be configured to only operate in the Automatic LOP mode or only operate in the Manual Pressure Mode.

Referring again to FIG. 4, after power button **240** is pressed on housing **210** of motor/pump unit **200,** an optional Welcome Screen is displayed on display **230**. Also, while the Welcome Screen is displayed on display **230,** motor/pump unit **200** can optionally be configured to conduct an ambient air pressure calibration with the one or more pressure sensors in housing **210** of motor/pump unit **200**.

After the optional Welcome Screen, an optional Warning Screen and/or Terms Of Use Screen can be displayed. One or more buttons **250, 260** may optionally be required to be pressed or display screen **230** touched (when display is a touch screen display) to indicate acknowledgement of the warnings and/or Terms of Use.

After the optional Warning Screen and/or Terms Of Use Screen, if motor/pump unit **200** has more than one mode, then the two or more modes of operation of motor/pump unit **200** can be displayed so that the user can select the mode of operation by use of one or more buttons **250, 260** or by touching display screen **230** (when display is a touch screen display). As illustrated in FIG. 4, motor/pump unit **200** has four (4) operational modes (e.g., Automatic LOP mode, Manual Pressure mode, IPC mode, PEIC mode). FIG. 1 illustrates the operation of the BFRT cuff **100** and motor/pump unit **200** when the IPC mode (Ischemic Preconditioning mode) or PEIC mode (Post Exercise Ischemic Conditioning mode) are selected by the user.

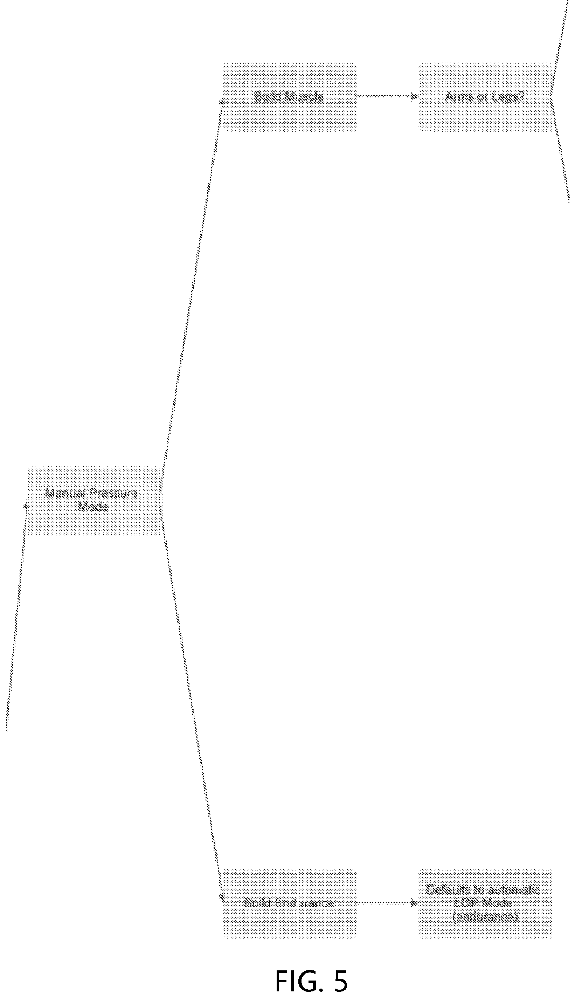

When the Manual Pressure mode is selected by the user, the operation of BFRT cuff **100** and motor/pump unit **200** will follow the operation illustrated in FIGS 5-7. When motor/pump unit **200** only has a single Manual Pressure Mode or motor/pump unit **200** does not have an Automatic LOP mode and the IPC or PEIC modes are not selected, the operation of BFRT cuff **100** and motor/pump unit **200** can optionally be configured to follow the operation illustrated in FIGS 5-7 without having the user select another mode.

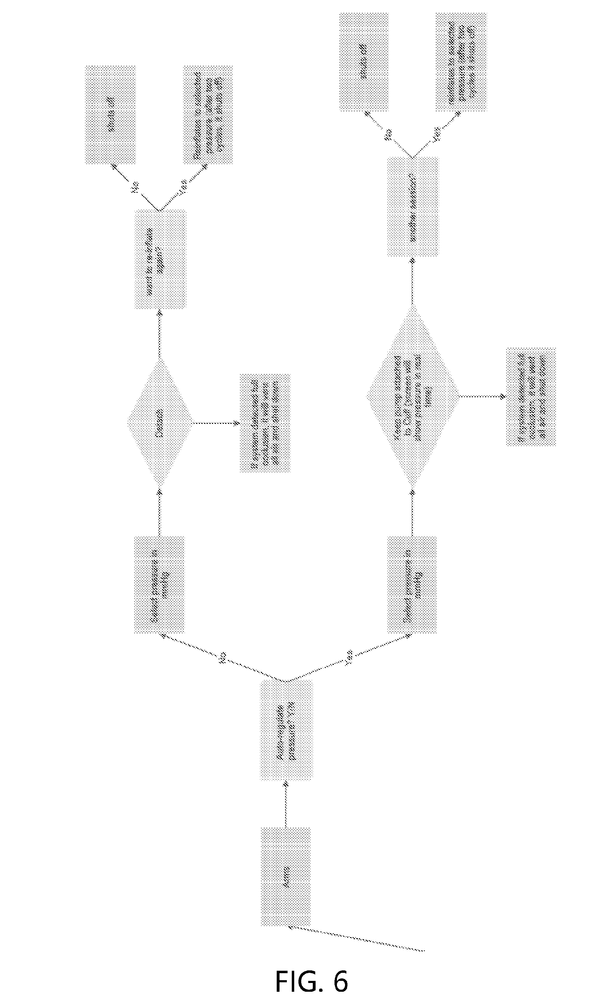

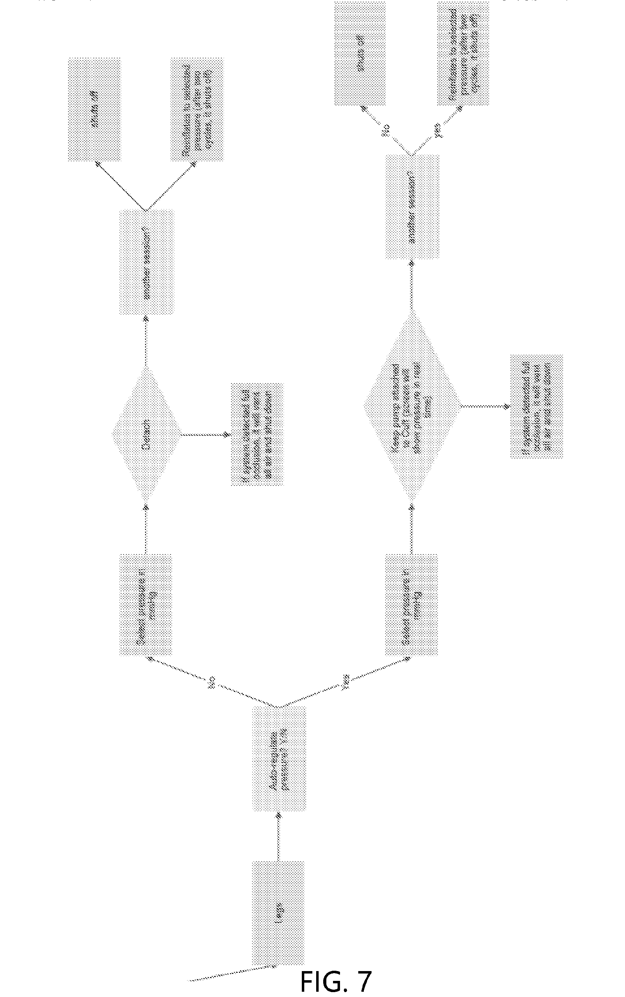

FIG. 5 illustrates that the user can select between building muscle or building endurance. However, if BFRT cuff **100** and motor/pump unit **200** does not have an Automatic LOP mode, then only the build muscle option can be used by the user. When the build muscle option is selected, the user is optionally asked to select whether the arm or leg is to have BFRT cuff attached thereto. FIG. 6 illustrates the operation of BFRT cuff **100** and motor/pump unit **200** when the arm is selected, and FIG. 7 illustrates the operation of BFRT cuff **100** and motor/pump unit **200** when the leg is selected.

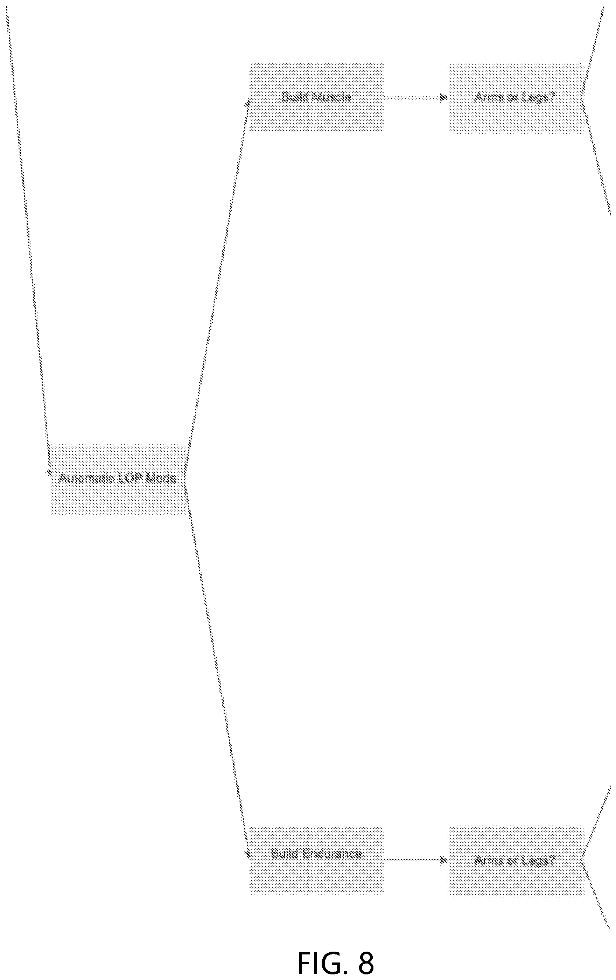

When BFRT cuff **100** and motor/pump unit **200** have an Automatic LOP mode, FIGS. 8-12 illustrated the operation of BFRT cuff **100** and motor/pump unit **200** is such mode.

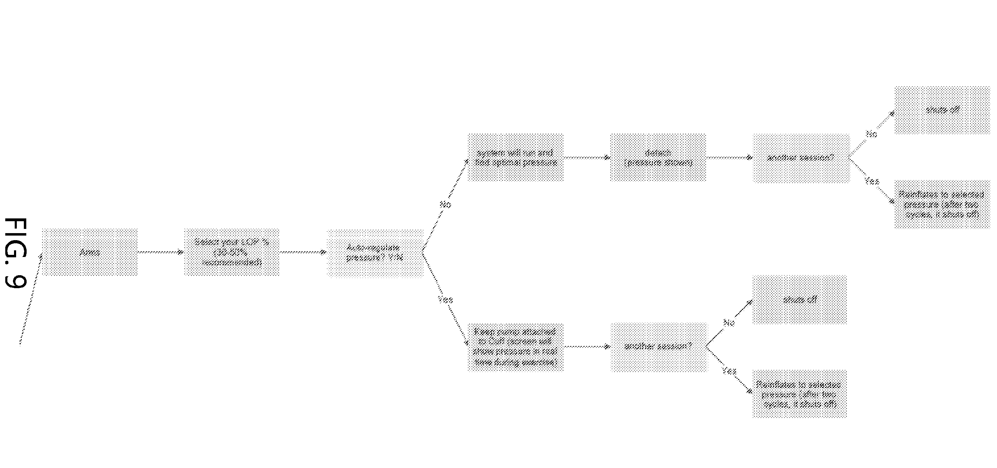

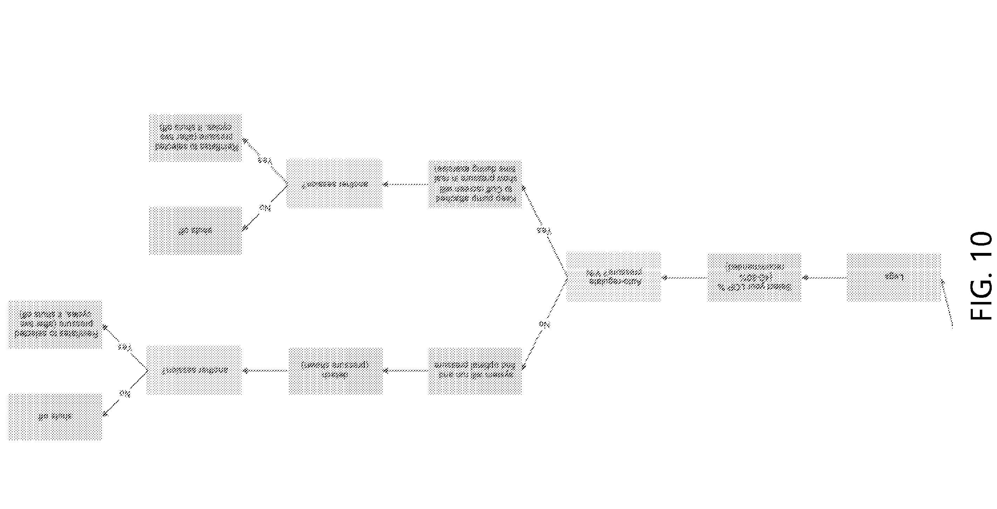

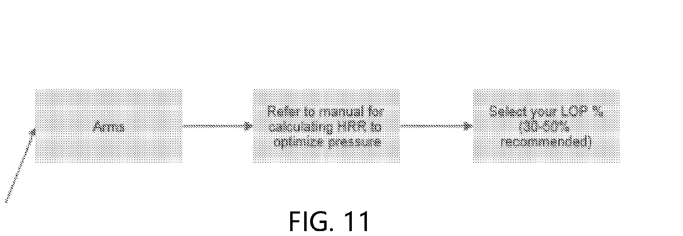

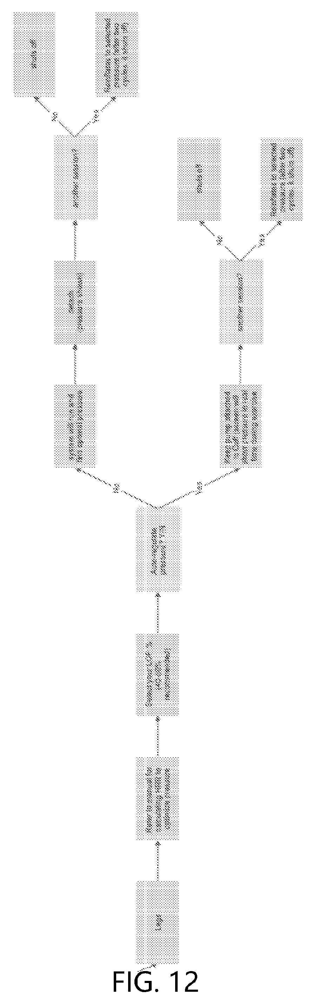

FIG. 8 illustrates that the user can optionally be asked to select the build muscle mode or the build endurance mode. After either mode is selected, the user is then optionally asked to select whether the arm or leg is to have BFRT cuff **100** attached thereto. FIG. 9 illustrates the operation of BFRT cuff **100** and motor/pump unit **200** when the arm is selected in the build muscle mode, FIG. 10 illustrates the operation of BFRT cuff **100** and motor/pump unit **200** when the leg is selected in the build muscle mode. FIG. 11 illustrates the operation of BFRT cuff **100** and motor/pump unit **200** when the arm is selected in the build endurance mode, and FIG. 12 illustrates the operation of BFRT cuff **100** and motor/pump unit **200** when the leg is selected in the build endurance mode.

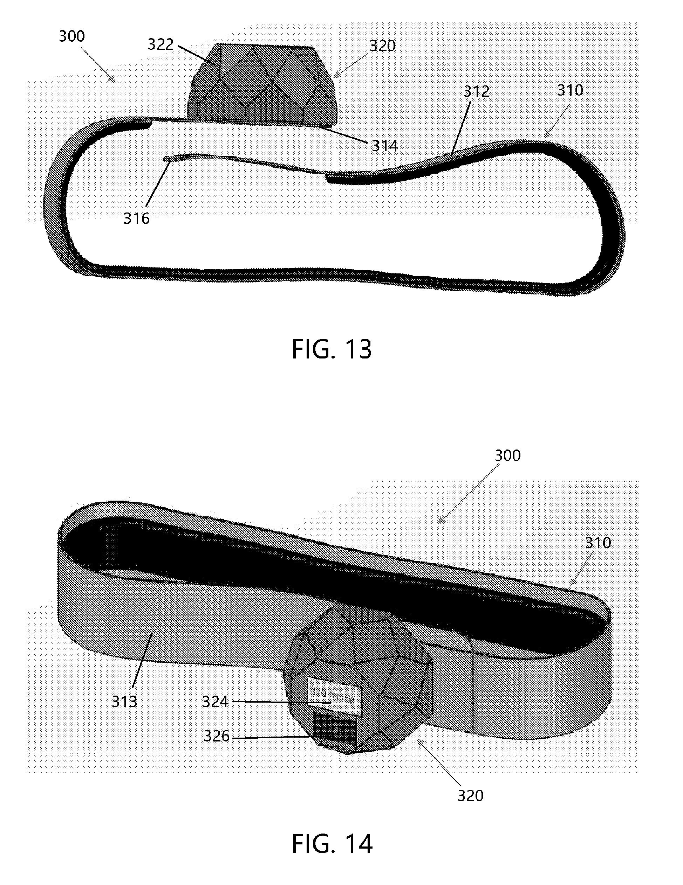

Referring now to FIGS. 13-17, an alternative non-limiting BFRT system **300** is illustrated that includes a BFRT cuff **310** and motor/pump unit **320**.

BFRT cuff **310** includes an inflatable air system formed of at least two layers of material coupled together to create one or more air chambers to be inflated with air and/or some other gas. The inflatable air system is configured in BFRT cuff **310** such that inflation of the inflatable air system produces compression on a target compression zone that in turn produces a restriction of blood flow in the venous system of a user. The BFRT cuff **310** includes an outer material cover **312** having a cavity for containing the inflatable air system so as to isolate the inflatable air system from the skin of the user. The inflatable air system can optionally be secured in the cavity to one or more inner surface of the outer material cover (e.g., stitching, adhesive, melted connection, etc.). The outer material cover **312** is generally made of a flexible and durable material (e.g., nylon, Kevlar^{™}, etc.). In one non-limiting embodiment, outer material cover **312** is formed of an inelastic or non-stretch material. Outer material cover **312** is configured to a) facilitate in distributing the force applied by the inflatable air system to the limb of a user, b) reduce pinching of the user's skin during the inflation of the inflatable air system, and/or c) provide friction between the BFRT and the user's skin to inhibit or prevent rotation of BFRT cuff 310 on the user during use.

BFRT cuff **310** includes one or more air input ports **318** in fluid communication with the inflatable air system to allow a fluid (e.g., air) to flow into and out of motor/pump unit **320**. Input port **318** generally includes a valve component. The location of the one or more input ports **318** on BFRT cuff **310** is non-limiting. Generally, at least one input port **318** is located on an outwardly facing surface of outer material cover **312.** One or more of input ports **318** is configured to be connected to a motor/pump unit **320**. One or more input ports **318** can optionally include a one-way valve (not shown) to prevent undesired deflation of the air inflatable system. The one or more input ports **318** can optionally be configured to allow for manual deflation of the air inflatable system.

As illustrated in FIGS. 13-17, BFRT cuff **310** has a motor/pump unit **320** connected to an outer surface **313** of outer material cover **312.** In particular, outer material cover **312** includes a first end **314** and second end **316** and motor/pump unit **320** is connected to a portion of outer material cover **312** that is located closest to first end **314.** As can be appreciated, motor/pump unit **320** can be located on any portion of outer material cover **310.** Although not shown, the outer material cover **310** is connected to an input port **318** so that motor/pump unit **320** can inflate/deflate the air inflatable system (not shown) that is located in the cavity (not shown) of outer material cover **310.**

Motor/pump unit **320** includes a) a housing **322;** b) a motor (not shown); c) a pump (not shown); d) one or more displays **324** (e.g., LED screen, OLED screen, etc.) to display measured pressure and/or to display other information; e) optional one or more processors (not shown); f) optional wireless electronics (not shown) that can be used to send/receive wireless signals between the motor/pump unit and a computer, server, data hub, cloud, tablet, smart phone and/or other type of smart device; g) one or more pressure gauges and/or sensors (not shown) used to measure a gas pressure in the air inflatable system; h) optional power source (not shown) to power one or more components of motor/pump unit **320** (e.g., motor, pump, pressure sensor/gauge; electronics, display, etc.); i) optional one or more power ports (not shown) to charge the power source; j) optional connection arrangement **330** to permanently or releasably connect motor/pump unit **320** to outer material cover **310;** k) optional one or more fluid connectors **330** to fluidly connect motor/pump unit **320** to the air inflatable system; l) optional GPS system (not shown); m) optional circuit board and other electronics (not shown); n) optional software (not shown); o) optional one or more data ports (e.g., micro USB port, etc.) (not shown) to enable wired transfer of data between motor/pump unit **320** and a computer, server, data hub, cloud, tablet, smart phone and/or other type of smart device; p) optional one or more buttons (e.g., power button, etc.) (not shown), r) optional one or more selection or scroll buttons (not shown) to enable manual operation/control of motor/pump unit **320** and/or to select certain information to be displayed on motor/pump unit **320**; s) optional one or more switch(es) (not shown), to enable manual operation/control of motor/pump unit **320**; r) optional air hose port (not shown); t) optional one or more lights and/or other indicators (not shown) to indicate a mode and/or operation and/or status of motor/pump unit **320**; and u) optional memory (not shown). The shape, size and weight of housing **322** is non-limiting. The material used to form the housing is generally a durable material (e.g., plastic, metal, composite, etc.). The size and weight of motor/pump unit **320** can be the same or similar to motor/pump unit **200** as described above.

As can be appreciated, motor/pump unit **320** can be partially of fully contained within BFRT cuff **310** and/or partially or fully contained within the air inflatable system of BFRT cuff **310.** In such an arrangement, motor/pump unit **320** can be partially or fully hidden within BFRT cuff **310.** In such an arrangement, outer surface **313** of outer material cover **312** can optionally include one or more displays and/or buttons to display information regarding motor/pump unit **320,** display information regarding pressure of the air inflation system, and/or to manually control motor/pump unit **320**. As can be appreciated, the one or more displays and/or buttons can be used to display information and/or control motor/pump unit **320** as described above with regard to motor/pump unit **200**. In such an arrangement, housing **320** of FIG. 13 merely represents a display **326** and optional buttons on outer surface **313** of outer material cover **312** and some or all of the other components of motor/pump unit **200** are located within the air inflatable system of the BFRT cuff **310.**

As illustrated in FIG. 14, front surface **324** of motor/pump unit **320** includes a LED display **326** that displays the measured air pressure in the air inflatable system. As can be appreciated, other and/or additional information can be displayed on display **326.** The front surface **324** can optionally include one or more buttons and/or switches (not shown).

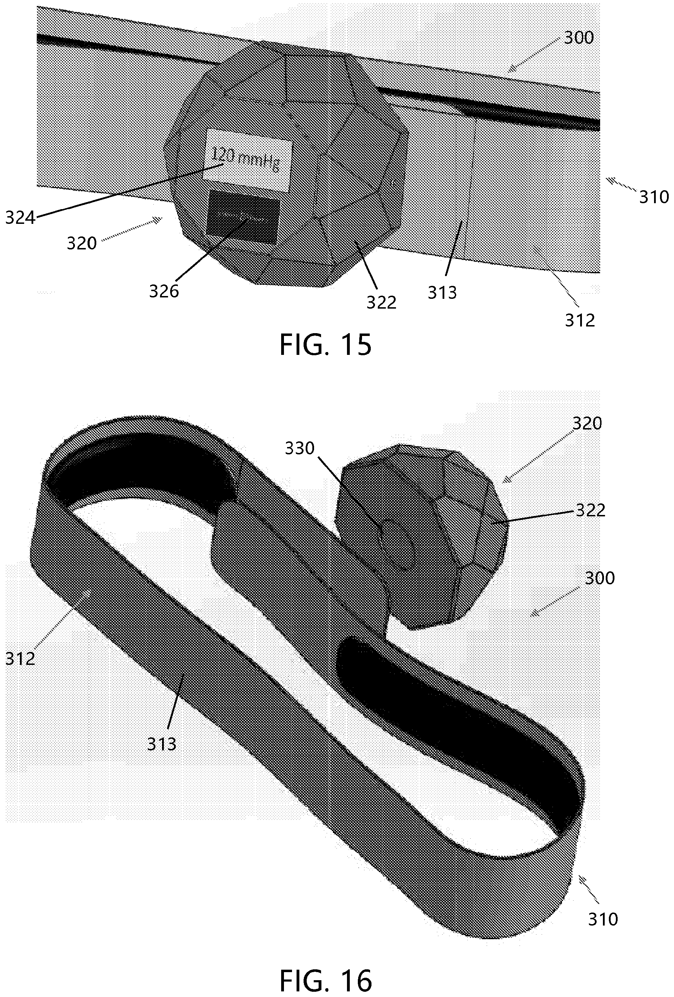

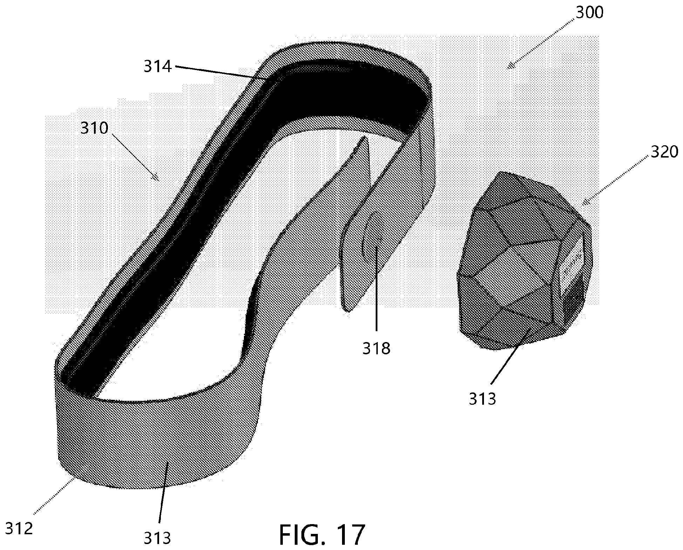

Referring now to FIGS. 15-16, motor/pump unit **320** is illustrated as being releasably connectable to outer surface **313** of outer material cover **312** of BFRT cuff **310.** The type of connection arrangement is non-limiting (e.g., snap connection arrangement, hook and loop fastener, slot connection arrangement, tongue and groove connector, etc.). As illustrated in FIG. 17, outer surface **313** of outer material cover **312** of BFRT cuff **310** includes a connector **318** configured to releasably connect to housing **322** of motor/pump unit **320**.

BFRT cuff **310** includes a cuff fastening arrangement (not shown) to releasably secure BFRT cuff **310** to a user. The type of fastening arrangement is non-limiting (e.g., snap connection arrangement, hook and loop fastener, snap fastener, button fastener, etc.).

### Operation of BFRT System

The operation of BFRT system **300** can be the same of similar to the operation as described above with regard to BFRT system **20**.

Another non-limiting operation sequence of BFRT system **300** is set forth below. It will be understood that one or more steps described above with regard to BFRT system **20** can be used with the one or more step of BFRT system **300** described below.

In the first step, a user (not shown) selects an appropriate size of BFRT cuff **310** based on the user's upper arm girth if exercising the upper limbs, or upper thigh girth if exercising the lower limbs.

In the next step, the user applies BFRT cuff **310** about his/her arm or leg and secures BFRT cuff **310** in the desired position on his/her arm or leg. Motor/pump unit **320** should be connected to BFRT cuff **310** prior to attempting to inflate the air inflatable system of BFRT cuff **310.** In some non-limiting embodiments, motor/pump unit **320** is integrated in BFRT cuff **310,** thus does not need to be connected to BFRT cuff **310.**

The next step is to inflate the air inflatable system in BFRT cuff **310.** This can be accomplished by manually activating motor/pump unit **320,** or by wirelessly activating motor/pump unit **320**. Such wireless activation can be accomplished by using a mobile app or program on a smart device (e.g., smart phone, tablet, computer, laptop computer, server, data hub, cloud, etc.) and the like. As can be appreciated, a smart device and the like can also or alternatively be wired connected to motor/pump unit **320** or BFRT cuff **310** (when motor/pump unit **320** is integrated in BFRT cuff **310).**

The pump in motor/pump unit **320** injects air into the air inflatable system in BFRT cuff **310** until a desired pressure is reached. The pressure in the air inflatable system can be optionally viewed on display **324** on housing **322** of motor/pump unit **320** and/or on another device (e.g., smart phone, computer, tablet, etc.). BFRT cuff **310** can optionally include a pressure limiting valve (not shown) to prevent over pressurization of the air inflatable system. Once the desired pressure is achieved in the air inflatable system, the operation of the pump is terminated. Thereafter, the user can proceed to exercise.

During exercise, the BFRT system can include an Auto Regulation Feature whereby if the pressure sensor determines that the pressure in the air inflatable system has exceed a predetermined pressure, air pressure can be released from the air inflatable system until the predetermined pressure in the air inflatable system is obtained. If the pressure sensor determines that the pressure in the air inflatable system is below a predetermined pressure, air pressure can be increased in the air inflatable system by activating motor/pump unit **320** until the predetermined pressure in the air inflatable system is obtained. The increase/decrease in pressure in the air inflatable system can occur during the use of BFRT cuff **310** without requiring the user to interrupt his/her exercise. Such a feature is a significant improvement over prior art BFRT arrangements wherein a user needed to stop exercising to manually increase or decrease the air pressure in the BFRT arrangement.

As illustrated in FIGS. 16-17, motor/pump unit **320** can be configured to be removable from BFRT cuff **310.** If motor/pump unit **320** is removed from BFRT cuff **310** after the air inflatable system has been inflated, BFRT cuff **310** typically includes a valve arrangement that prevents deflation of the air inflatable system during and after the removal of motor/pump unit **320** from BFRT cuff **310.**

When the user is through with the desired exercises, the user may manually release the pressure in the air inflatable system, use an app or program on a computer or other type of smart device to deflate the air inflatable system, and/or the air inflatable system can be automatically deflated. Thereafter, the user can remove BFRT cuff **310** from his/her arm or leg.

As can be appreciated, many of the steps of operation of BFRT cuff **310** can be the same or similar to the steps of operation of BFRT cuff **100** as described above.

BFRT cuff **310** of the present disclosure includes several unique features as discussed in more detail below.

BFRT cuff **310** can have a urethane-coated air bladder. The urethane coating on the bladder adds to patient comfort by reducing pinching of the skin by the bladder when inflated.

BFRT cuff **310** includes a motor/pump unit **320** to inflate/deflate the one or more air chambers in BFRT cuff **310,** and which motor/pump unit **320** is detachable connectable to BFRT cuff **310** and can remain connected to BFRT cuff or be removed from the BFRT cuff prior to, during, and/or after use of BFRT cuff **310.** When motor/pump unit **320** remains on BFRT cuff **310** while the user exercises, motor/pump unit **320** can be configured to automatically activate to reduce or increase air pressure in the air inflatable system so as to maintain a desired air pressure in the air inflatable system during the exercise routine. Alternatively, the user can manually activate motor/pump unit **320** to increase/decrease the pressure in the air inflatable system. Motor/pump unit **320** can be removed from BFRT cuff motor/pump unit **320** to reduce the weight of BFRT system **300** on the user during exercise or to prevent inadvertent damage to motor/pump unit **320** during rigorous exercise. Motor/pump unit **320** can be connected to and detached from BFRT cuff **310** at any time prior to, during, or after an exercise routine. BFRT cuff **310** includes a valve arrangement that prevents undesired deflation of the air inflatable system during the connection and detachment of motor/pump unit **320** to/from BFRT cuff **310.**

Motor/pump unit **320** can be powered by a battery contained in the housing of motor/pump unit **320**. Such a feature eliminates the need for power cords to operate motor/pump unit **320,** thus providing more flexibility and ease of use of BFRT cuff **310.** The battery can be rechargeable and housing **322** of motor/pump unit **320** can optionally include a power port so that a power cord, USB cord, etc. can be connected to housing **322** to recharge the battery. As can be appreciated, motor/pump unit **320** can be designed to allow for wireless charging of the battery.

Motor/pump unit **320** used with BFRT cuff **310** can include one or more displays **324.** These one or more displays **324** can be used to inform a user of a) whether the pump is on/off, b) battery status, c) pressure level for pressure in the one or more air chambers of BFRT cuff **310,** d) time/date, e) whether pressure in the one of more air chambers of BFRT cuff **310** is at or below limb occlusion pressure, f) error notification/status, g) security status (e.g., locked, unlocked, etc.), h) ambient temperature, i) timer/clock information, j) alarm status/notification, k) time of use, l) pressure mode, operation mode, and/or selected training session, m) whether full occlusion has been obtained, n) whether maximum workout time period has occurred, and/or o) wireless connection status to computer, tablet, smart phone, and/or other type of smart device.

Motor/pump unit **320** on BFRT cuff **310** can be configured to be manually controlled by the user activating/pressing/moving buttons, switches, locations on the display, etc., and/or motor/pump unit **320** can be configured to be controlled wirelessly and/or wired by a computer, tablet, smart phone, and/or other type of smart device. The wired and/or wireless control of motor/pump unit **320** can be accomplished via a program or app running on a computer, tablet, smart phone, and/or other type of smart device. Wired and/or wireless control of motor/pump unit **320** can also be accomplished by voice commands. As can be appreciated, motor/pump unit **320** can include electronics, software, etc. to enable voice control of motor/pump unit **320** without the need of a computer or other type of smart device. When programs or apps are used to wire and/or wirelessly control motor/pump unit **320,** these programs or apps can be used to both monitor the operation of motor/pump unit **320** as well as control the operation of motor/pump unit **320** and/or monitor the status of BFRT cuff **310.**

BFRT cuff **310** can optionally include a pressure sensor and one or more processors to a) facilitate in controlling/maintaining/regulating the pressure in the one or more air chambers of BFRT cuff **310,** b) provide pressure information to motor/pump unit **320** to be displayed on motor/pump unit **320** and/or wired and/or wirelessly sent to a remote device, c) provide pressure information to be displayed on one or more displays on BFRT cuff **310** and/or wired and/or wirelessly sent to a remote device, d) provide pressure information wirelessly to a computer, tablet, smart phone, and/or other type of smart device and have such information displayed using a computer program or mobile app. The one or more pressure sensors can be part of the motor/pump unit **320** and/or be located at a different location on BFRT cuff **310.**

Motor/pump unit **320** on BFRT cuff **310** can be configured to allow a user to manually input a predetermined limb occlusion pressure; motor/pump unit **320** uses such information to control/maintain/regulate the pressure in the one or more air chambers of BFRT cuff **310.** BFRT cuff **310** can also or alternatively be configured to wired and/or wirelessly receive information regarding the automatic calculation of a predetermined limb occlusion pressure via a computer program or mobile app running on a computer, tablet, smart phone, and/or other type of smart device and to then use such information to control/maintain/regulate the pressure in the one or more air chambers of BFRT cuff **310.**

BFRT cuff **310** can include one or more sensors and/or control systems to automatically shut off the pump and/or release pressure in the one or more air chambers of BFRT cuff **310** after sensing that an occlusion pressure in the one or more air chambers has been maintained for some preset period of time (e.g., 0.001-30 seconds, 0.001 seconds to 1 minute, 0.001 seconds to 4 minutes, 0.001 seconds to 5 minutes, etc.). In one non-limiting arrangement, BFRT cuff **310** and/or motor/pump unit **320** can include one or more sensors and/or control systems to automatically shut off the pump and/or release pressure in the one or more air chambers of BFRT cuff **310** after sensing that an occlusion pressure in the one or more air chambers has been maintained for 0.001 seconds to 45 seconds (and all values and ranges therebetween). Such a feature facilitates in the proper use of BFRT cuff **310** and prevents extended periods of time of occlusion pressure being applied to user so as to not damage or impair rehabilitation of the tissue of the user during the use of BFRT cuff **310.**

BFRT cuff **310** and/or motor/pump unit **320** can include a sensor and/or control system to automatically shut off the motor/pump unit and/or to release pressure in the one or more air chambers of BFRT cuff **310** after some preset period of time (e.g., 20 minutes, etc.). Such a feature preserves the life of BFRT cuff **310** and functions as another safety feature of BFRT cuff **310.** In one non-limiting arrangement, BFRT cuff **310** and/or motor/pump unit **320** include one or more sensors and/or control systems to automatically shut off pump and/or motor/pump unit **320,** and/or to release pressure in the one or more air chambers of BFRT cuff **310** after 2-20 minutes (and all values and ranges therebetween).

BFRT cuff **310** and/or motor/pump unit **320** can include one or more sensors to detect use and non-use of BFRT cuff **310** (e.g., motion sensor, etc.) and to automatically shut off the pump and/or releases pressure in the one or more air chambers of BFRT cuff **310** after some preset period of time of non-use of BFRT cuff **310** (e.g., 1-20 minutes, etc.). Such a feature preserves the life of BFRT cuff **310.** In one non-limiting arrangement, BFRT cuff **310** and/or motor/pump unit **320** include one or more sensors to detect use and non-use of BFRT cuff **310** and to automatically shut off the pump and/or releases pressure in the one or more air chambers of BFRT cuff **310** after 1-10 minutes (and all values and ranges therebetween) of non-use of BFRT cuff **310.**

BFRT cuff **310** can optionally include a GPS sensor or other type of location sensor. This type of sensor is used to track the location of BFRT cuff **310** and/or to monitor movement of BFRT cuff **310** during an exercise routine to determine if proper use of BFRT cuff **310** is being utilized by the user during a certain exercise routine.

BFRT cuff **310** can optionally include one or more lateral sensors, movement sensors and/or a gyroscope arrangement to monitor movement of BFRT cuff **310** during an exercise routine to determine if proper use of BFRT cuff **310** is being utilized by the user during a certain exercise routine.

BFRT cuff **310** can include one or more pressure modes (e.g., ischemic preconditioning mode ("IPC"), manual mode, auto mode, etc.). These modes can be manually selected, wirelessly selected, wire selected, and/or selected by voice command. The IPC mode is a mode in which BFRT cuff **310** is inflated to full occlusion of the artery for some time period (e.g., 10 seconds-5 minutes [and all values and ranges therebetween], etc.) and at some rest period interval (e.g., 10 seconds-4 minutes [and all values and ranges therebetween], etc.) between the periods of full occlusion. In auto mode, motor/pump unit **320** automatically sets the optimal pressure based on what LOP % set by the user or by information wirelessly received from a device that has automatically calculated the pressure optimal pressure based on what LOP % that is set by the user. In the manual mode, the user manually sets the pressures on BFRT cuff **310** or on a device that then wired and/or wirelessly sends the pressure information to BFRT cuff **310.**

BFRT cuff **310** can be monitored and/or controlled by a computer program and/or mobile app running on a computer, tablet, smart phone, and/or other type of smart device. The computer program and/or mobile app can 1) select a mode of operation for BFRT cuff **310,** 2) activate/deactivate BFRT cuff **310,** 3) transmit/receive information to/from BFRT cuff **310,** 4) select/control/maintain/reduce the pressure in the one or more air chambers of BFRT cuff **310** while the user uses BFRT cuff **310,** 5) monitor the operation of BFRT cuff **310,** 6) maintain a log regarding the use of BFRT cuff **310,** 7) provide an exercise plan for a user that is using BFRT cuff **310,** 8) record/monitor the progress/compliance of a user's exercise plan, 9) select/recommend a BFRT training session for a user, 10) keep track of a user's BFRT training sessions, 10) provide suggested training tips for BFRT to the user, 11) provide BFRT information to a user, 12) transmit/receive data to/from a user's BFRT trainer or instructor, 13) maintained a pressure or pressure range or % LOP or range of % LOP in the BFRT cuff during use, and/or 14) partially or fully deflate the BFRT cuff for a certain period of time after an exercise is completed, between exercise routines, or after non-use of the BFRT cuff is detected.

BFRT cuff **310** can include security protocols to prevent misuse and/or unauthorized use of BFRT cuff **310.** A computer program or mobile app that can run on a computer, tablet, smart phone, and/or other type of smart device can include password protection and/or other security controls to prevent unauthorized use of BFRT cuff **310** and/or access to personal information regarding the use of BFRT cuff **310**. The motor/pump unit **320** can also include password protection to prevent unauthorized operation of BFRT cuff **310.**

To aid the Patent Office and any readers of this application and any resulting patent in interpreting the claims appended hereto, Applicant does not intend any of the appended claims or claim elements to invoke 35 U.S.C. 112(f) unless the words "means for" or "step for" are explicitly used in the particular claim.

It will thus be seen that the objects set forth above, among those made apparent from the preceding description, are efficiently attained, and since certain changes may be made in the constructions set forth without departing from the spirit and scope of the invention, it is intended that all matter contained in the above description and shown in the accompanying drawings shall be interpreted as illustrative and not in a limiting sense. The invention has been described with reference to preferred and alternate embodiments. Modifications and alterations will become apparent to those skilled in the art upon reading and understanding the detailed discussion of the invention provided herein. This invention is intended to include all such modifications and alterations insofar as they come within the scope of the present invention. It is also to be understood that the following claims are intended to cover all of the generic and specific features of the invention herein described and all statements of the scope of the invention, which, as a matter of language, might be said to fall there between. The invention has been described with reference to the preferred embodiments. These and other modifications of the preferred embodiments as well as other embodiments of the invention will be obvious from the disclosure herein, whereby the foregoing descriptive matter is to be interpreted merely as illustrative of the invention and not as a limitation. It is intended to include all such modifications and alterations insofar as they come within the scope of the appended claims.

### Reference Numbers

- 20: BFRT system
- 100: BFRT cuff
- 102: outer material cover
- 110: outer material cover
- 112: outer surface
- 114: first end
- 116: second end
- 120: strap
- 130: input ports
- 200: motor/pump unit
- 210: housing
- 220: data port
- 230: display
- 240: button
- 241: button
- 250: button
- 260: button
- 270: air hose
- 280: connection arrangement
- 290: hose port
- 292: battery charge level
- 294: warning
- 300: BFRT system
- 310: BFRT cuff
- 312: outer material cover
- 313: outer surface
- 314: first end
- 316: second end
- 318: input ports
- 320: motor/pump unit
- 322: housing
- 324: display
- 326: display
- 330: fluid connectors

## Claims

1. A method for exercising using a blood flow restriction training (BFRT) system (20) comprising:
a. providing a BFRT cuff (100), said BFRT cuff (100) including i) a flexible outer material, ii) an air chamber at least partially encapsulated by said flexible outer material, and iii) a connection arrangement (280) configured to secure said BFRT cuff (100) to a limb of a user, said flexible outer material having a length of at least 20 cm and a width of at least 5 cm;
b. providing a motor/pump unit (200), said motor/pump unit (200) configured to at least partially inflate said BFRT cuff (100), said motor/pump unit (200) including a housing (210) and an air connection arrangement (280) that fluidly connects said housing (210) to said BFRT cuff (100) to enable pumped air from said motor/pump unit (200) to flow to and at least partially inflate said air chamber, said housing (210) having a size less than 6.55 × 10³ cm³ (400 cubic inches) and a weight less than 1.81 kg (4 lbs); said housing (210) at least partially containing i) an air pump, ii) a power source, and iii) a pressure sensor;
c. placing said BFRT cuff (100) on a user's limb;
d. connecting said BFRT cuff (100) to said motor/pump unit (200) to enable air flow between said BFRT cuff (100) and said motor/pump unit (200);
e. selecting a select pressure value or percent of limb occlusion pressure (LOP) based on said user's limb which said BFRT cuff (100) is placed, said pressure value less than said LOP, said percent no more than 90%;
f. causing said motor/pump unit (200) to supply air to said BFRT cuff (100) to thereby cause said BFRT cuff (100) to inflate until said LOP of said limb is obtained;
g. terminating air flow from said motor/pump unit (200) to said BFRT cuff (100) after said LOP has been obtained for the limb;
h. reducing pressure in said BFRT cuff (100) within 0.001-30 seconds after said LOP is obtained in said BFRT cuff (100) until said pressure value is said select pressure value or said percent of LOP; and,
i. exercising the user's limb while said BFRT cuff (100) is positioned on said limb of said user; and wherein A) said motor/pump unit activates said air pump when said pressure in said air chamber falls below said selected pressure value, falls below said percent of said LOP, falls to or below a preset minimum pressure value, or falls to or below a preset minimum percent LOP value during said step of exercising to increase said pressure in said air chamber, and/or B) said motor/pump unit (200) or said BFRT cuff (100) causes said pressure in said air chamber to be reduced when said pressure in said air chamber exceeds below said selected pressure value, exceeds said percent of said LOP, obtains or exceeds a preset maximum pressure value, or obtains or exceed a preset maximum percent LOP value during said step of exercising so as to decrease said pressure in said air chamber;
**characterized by**
automatically selecting the select pressure value or percent of limb occlusion pressure (LOP) based on an input by said user of the limb upon which said BFRT cuff (100) is placed.

2. The method as defined in claim 1, wherein said step of reducing is at least partially accomplished by causing said motor/pump unit (200) to remove air from said BFRT cuff (100).

3. The method as defined in claim 1, further including the step of removing said BFRT cuff (100) from said limb after said step of exercising said user's limb.

4. The method as defined in claim 2, further including the step of removing said BFRT cuff (100) from said limb after said step of exercising said user's limb.

5. The method as defined in any one of claims 1-4, wherein said housing (210) of said motor/pump unit (200) provides three or more types of information selected from the group consisting of a) measured pressure, b) power or charge level of said power source, c) a warning symbol when some type of warning event has occurred, d) selected % LOP, e) selected pressure, f) instructional information as to how to use said motor/pump unit and/or BFRT cuff (100), and g) arm or leg selection options for BFRT cuff (100).

6. The method as defined in any one of claims 1-5, further including the step of turning on or powering up said motor/pump unit (200), said motor/pump unit (200) configured to calibrate with ambient pressure after said motor/pump unit (200) is turned on or powered up.

7. The method as defined in any one of claims 1-6, further including the step of selecting the limb upon which said motor/pump unit (200) that said BFRT cuff (100) is to be placed.

8. A method for exercising using a blood flow restriction training (BFRT) system comprising:
a. providing a BFRT cuff (100), said BFRT cuff (100) including i) an air chamber at least partially encapsulated by said flexible outer material, and ii) a connection arrangement configured to secure said BFRT cuff (100) to a user's limb;
b. providing a motor/pump unit (200), said motor/pump unit (200) configured to at least partially inflate said BFRT cuff (100), said motor/pump unit (200) including a housing (210) and an air connection arrangement (280) that fluidly connects said housing (210) to said BFRT cuff (100) to enable pumped air from said motor/pump unit (200) to flow to and at least partially inflate said air chamber, said housing (210) at least partially containing i) an air pump, and ii) a power source;
c. placing said BFRT cuff (100) on a user's limb;
d. connecting said BFRT cuff (100) to said motor/pump unit (200) to enable air flow between said BFRT cuff (100) and said motor/pump unit (200);
e. selecting a pressure value or percent of limb occlusion pressure (LOP), said pressure value less than said LOP;
f. causing said motor/pump unit (200) to supply air to said BFRT cuff (100) to thereby cause said BFRT cuff (100) to inflate until said LOP of said limb is obtained;
g. terminating air flow from said motor/pump unit (200) to said BFRT cuff (100) after said LOP has been obtained for the limb;
h. reducing pressure in said BFRT cuff (100) after said LOP is obtained in said BFRT cuff (100) until said pressure value is said selected pressure value or said percent of said LOP; and,
i. exercising the limb of said user while said BFRT cuff (100) is positioned on said limb of said user; and
wherein A) said motor/pump unit (200) activates said air pump when said pressure in said air chamber falls below said selected pressure value, falls below said percent of said LOP, falls to or below a preset minimum pressure value, or falls to or below a preset minimum percent LOP value during said step of exercising so as to increase said pressure in said air chamber to be within certain pressure range or within a certain range of percent LOP, and/or B) said motor/pump unit (200) or said BFRT cuff (100) causes said pressure in said air chamber to be reduced when said pressure in said air chamber exceeds said selected pressure value, exceeds said percent of said LOP, obtains or exceeds a preset maximum pressure value, or obtains or exceed a preset maximum percent LOP value during said step of exercising to decrease said pressure in said air chamber to be within certain pressure range or within a certain range of percent LOP; and
wherein I) said motor/pump unit (200) or said BFRT cuff (100) causes said pressure in said air chamber to be reduced below said selected pressure value or said percent of said LOP when said motor/pump unit (200) or said BFRT cuff (100) detects inactivity of use of said BFRT cuff (100) by said user after a preselect period of time, or II) said motor/pump unit (200) or said BFRT cuff (100) causes said pressure in said air chamber to be reduced below said selected pressure value or said percent of said LOP after said motor/pump unit or said BFRT cuff (100) detects that a certain number of repetitions have been completed or a selected exercise has been completed;
**characterized by**
automatically selecting the select pressure value or percent of limb occlusion pressure (LOP) based on an input by said user of the limb upon which said BFRT cuff (100) is placed.

9. The method as defined in claim 8, including the step of wirelessly controlling said motor/pump unit.

10. A blood flow restriction training (BFRT) system comprising:
a. a BFRT cuff (100), said BFRT cuff (100) including i) an air chamber at least partially encapsulated by a flexible outer material, and ii) a connection arrangement (280) configured to secure said BFRT cuff to a user's limb; and
b. a motor/pump unit (200), said motor/pump unit (200) configured to at least partially inflate said BFRT cuff (100), said motor/pump unit (200) including a housing (210), and an air connection arrangement (280) that fluidly connects said housing (210) to said BFRT cuff (100) to enable pumped air from said motor/pump unit (200) to flow to and at least partially inflate said air chamber, said housing (210) at least partially containing i) an air pump, ii) a power source, and iii) a controller; said housing having a size less than 6.55 × 10³ cm³ (400 cubic inches) and a weight less than 1.81 kg (4 lbs); and
wherein said controller is configured to a) cause said motor/pump unit (200) to supply air to said BFRT cuff (100) to thereby cause said BFRT cuff (100) to inflate until said LOP of said limb is obtained; b) terminate air flow from said motor/pump unit (200) to said BFRT cuff (100) after said LOP has been obtained for the limb, c) reduce pressure in said BFRT cuff (100) after said LOP is obtained in said BFRT cuff (100) until a pressure value in said air chamber is at a selected pressure value or a selected percent of said LOP; and
wherein said controller is configured to A) activate said motor/pump unit (200) when said pressure in said air chamber falls below said selected pressure value, falls below said selected percent of said LOP, falls to or below a preset minimum pressure value, or falls to or below a preset minimum percent LOP value so as to increase said pressure in said air chamber to be within a certain pressure range or within a certain range of percent LOP, and/or B) activates said motor/pump unit (200) to cause said pressure in said air chamber to be reduced when said pressure in said air chamber exceeds said selected pressure value, exceeds said percent of said LOP, obtains or exceeds a preset maximum pressure value, or obtains or exceed a preset maximum percent LOP value so as to decrease said pressure in said air chamber to be within certain pressure range or within a certain range of percent LOP;
**characterized in that**
said controller is configured to automatically select the select pressure value or percent of limb occlusion pressure (LOP) based on an input by said user of the limb upon which said BFRT cuff (100) is placed.

11. The BFRT system as defined in claim 10, wherein said controller is configured to I) cause said motor/pump unit (200) to reduce said pressure in said air chamber below said selected pressure value or said percent of said LOP when said motor/pump unit (200) or said BFRT cuff (100) detects inactivity of use of said BFRT cuff after a preselect period of time, or II) cause said motor/pump unit (200) to reduce said pressure in said air chamber below said selected pressure value or said percent of said LOP when said motor/pump unit (200) or said BFRT cuff (100) detects that a certain number of repetitions have been completed or a selected exercise has been completed.

## Patentansprüche

1. Verfahren zum Trainieren unter Verwendung eines Blutflussrestriktionstrainingssystems (BFRT) (20), umfassend:
a. Bereitstellen einer BFRT-Manschette (100), wobei die BFRT-Manschette (100) i) ein flexibles Außenmaterial, ii) eine zumindest teilweise von dem flexiblen Außenmaterial umschlossene Luftkammer und iii) eine Verbindungsanordnung (280), die so konfiguriert ist, dass sie die BFRT-Manschette (100) an einem Gliedmaß eines Benutzers befestigt, wobei das flexible Außenmaterial eine Länge von mindestens 20 cm und eine Breite von mindestens 5 cm aufweist;
b. Bereitstellen einer Motor-/Pumpeneinheit (200), wobei die Motor-/Pumpeneinheit (200) so konfiguriert ist, dass sie die BFRT-Manschette (100) zumindest teilweise aufbläst, wobei die Motor-/Pumpeneinheit (200) ein Gehäuse (210) und eine Luftverbindungsanordnung (280) umfasst, die das Gehäuse (210) mit der BFRT-Manschette (100) verbindet, um zu ermöglichen, dass gepumpte Luft aus der Motor-/Pumpeneinheit (200) zu der Luftkammer strömt und diese zumindest teilweise aufbläst, wobei das Gehäuse (210) ein Volumen von weniger als 6,55 x 10³ cm³ (400 Kubikzoll) und ein Gewicht von weniger als 1,81 kg (4 lbs) aufweist; wobei das Gehäuse (210) zumindest teilweise i) eine Luftpumpe, ii) eine Energiequelle und iii) einen Drucksensor enthält;
c. Anlegen der BFRT-Manschette (100) an die Extremität eines Benutzers;
d. Verbinden der BFRT-Manschette (100) mit der Motor-/Pumpeneinheit (200), um einen Luftstrom zwischen der BFRT-Manschette (100) und der Motor-/Pumpeneinheit (200) zu ermöglichen;
e. Auswählen eines ausgewählten Druckwerts oder Prozentsatzes des Extremitätenokklusionsdrucks (LOP) basierend auf der Extremität des Benutzers, an der die BFRT-Manschette (100) angelegt ist, wobei der Druckwert kleiner als der LOP ist und der Prozentsatz nicht mehr als 90 % beträgt;
f. Veranlassen der Motor-/Pumpeneinheit (200), Luft an die BFRT-Manschette (100) zu liefern, um dadurch zu bewirken, dass sich die BFRT-Manschette (100) aufbläst, bis der LOP der Gliedmaße erreicht ist;
g. Beenden des Luftstroms von der Motor-/Pumpeneinheit (200) zur BFRT-Manschette (100), nachdem der LOP für die Extremität erreicht wurde;
h. Verringern des Drucks in der BFRT-Manschette (100) innerhalb von 0,001 bis 30 Sekunden, nachdem der LOP in der BFRT-Manschette (100) erreicht wurde, bis der Druckwert dem ausgewählten Druckwert oder dem Prozentsatz des LOP entspricht; und
i. Trainieren der Extremität des Benutzers, während die BFRT-Manschette (100) an der Extremität des Benutzers positioniert ist; und wobei A) die Motor-/Pumpeneinheit die Luftpumpe aktiviert, wenn der Druck in der Luftkammer während des Trainingsschritts zur Erhöhung des Drucks in der Luftkammer unter den ausgewählten Druckwert, unter den Prozentsatz des LOP, auf oder unter einen voreingestellten Mindestdruckwert oder auf oder unter einen voreingestellten Mindestprozentsatz des LOP fällt, und/oder B) die Motor-/Pumpeneinheit (200) oder die BFRT-Manschette (100) bewirkt, dass der Druck in der Luftkammer verringert wird, wenn der Druck in der Luftkammer während des Trainingsschritts den ausgewählten Druckwert überschreitet, den Prozentsatz des LOP überschreitet, einen voreingestellten maximalen Druckwert erreicht oder überschreitet oder einen voreingestellten maximalen LOP-Prozentsatzwert erreicht oder überschreitet, um den Druck in der Luftkammer zu verringern;
**gekennzeichnet durch**
automatisches Auswählen des ausgewählten Druckwerts oder Prozentsatzes des Extremitätenokklusionsdrucks (LOP) auf der Grundlage einer Eingabe durch den Benutzer der Extremität, an der die BFRT-Manschette (100) angelegt ist.

2. Das in Anspruch 1 definierte Verfahren, wobei der Schritt des Verringerns zumindest teilweise dadurch erreicht wird, dass die Motor-/Pumpeneinheit (200) veranlasst wird, Luft aus der BFRT-Manschette (100) abzulassen.

3. Das in Anspruch 1 definierte Verfahren, das ferner den Schritt des Entfernens der BFRT-Manschette (100) von der Extremität nach dem Schritt des Trainierens der Extremität des Benutzers umfasst.

4. Das in Anspruch 2 definierte Verfahren, das ferner den Schritt des Entfernens der BFRT-Manschette (100) von der Extremität nach dem Schritt des Trainierens der Extremität des Benutzers umfasst.

5. Das in einem der Ansprüche 1 bis 4 definierte Verfahren, wobei das Gehäuse (210) der Motor-/Pumpeneinheit (200) drei oder mehr Arten von Informationen bereitstellt, die aus der Gruppe ausgewählt sind, bestehend aus a) gemessenem Druck, b) Leistung oder Ladezustand der Stromquelle, c) einem Warnsymbol, wenn eine Art von Warnereignis aufgetreten ist, d) ausgewähltem % LOP, e) ausgewähltem Druck, f) Anweisungen zur Verwendung der Motor-/Pumpeneinheit und/oder der BFRT-Manschette (100) und g) Optionen zur Auswahl von Arm oder Bein für die BFRT-Manschette (100).

6. Das in einem der Ansprüche 1 bis 5 definierte Verfahren, das ferner den Schritt des Einschaltens oder Hochfahrens der Motor-/Pumpeneinheit (200) umfasst, wobei die Motor-/Pumpeneinheit (200) so konfiguriert ist, dass sie sich nach dem Einschalten oder Hochfahren der Motor-/Pumpeneinheit (200) auf den Umgebungsdruck kalibriert.

7. Verfahren nach einem der Ansprüche 1 bis 6, das ferner den Schritt der Auswahl der Extremität umfasst, an der die Motor-/Pumpeneinheit (200) mit der BFRT-Manschette (100) angebracht werden soll.

8. Verfahren zum Trainieren unter Verwendung eines Blutflussrestriktionstrainingssystems (BFRT), umfassend:
a. Bereitstellen einer BFRT-Manschette (100), wobei die BFRT-Manschette (100) i) eine Luftkammer, die zumindest teilweise von dem flexiblen Außenmaterial umschlossen ist, und ii) eine Verbindungsanordnung umfasst, die so konfiguriert ist, dass sie die BFRT-Manschette (100) an der Extremität eines Benutzers befestigt;
b. Bereitstellen einer Motor-/Pumpeneinheit (200), wobei die Motor-/Pumpeneinheit (200) so konfiguriert ist, dass sie die BFRT-Manschette (100) zumindest teilweise aufbläst, wobei die Motor-/Pumpeneinheit (200) ein Gehäuse (210) und eine Luftverbindungsanordnung (280) umfasst, die das Gehäuse (210) fluidisch mit der BFRT-Manschette (100) verbindet, um zu ermöglichen, dass gepumpte Luft aus der Motor-/Pumpeneinheit (200) zu der Luftkammer strömen und diese zumindest teilweise aufblasen kann, wobei das Gehäuse (210) zumindest teilweise i) eine Luftpumpe und ii) eine Energiequelle enthält;
c. Anlegen der BFRT-Manschette (100) an ein Gliedmaß eines Benutzers;
d. Verbinden der BFRT-Manschette (100) mit der Motor-/Pumpeneinheit (200), um einen Luftstrom zwischen der BFRT-Manschette (100) und der Motor-/Pumpeneinheit (200) zu ermöglichen;
e. Auswählen eines Druckwerts oder eines Prozentsatzes des Extremitätenokklusionsdrucks (LOP), wobei der Druckwert kleiner als der LOP ist;
f. Veranlassen, dass die Motor-/Pumpeneinheit (200) Luft an die BFRT-Manschette (100) liefert, um dadurch zu bewirken, dass sich die BFRT-Manschette (100) aufbläst, bis der LOP des Gliedes erreicht ist;
g. Unterbrechung des Luftstroms von der Motor-/Pumpeneinheit (200) zur BFRT-Manschette (100), nachdem der LOP für die Extremität erreicht wurde;
h. Reduzieren des Drucks in der BFRT-Manschette (100), nachdem der LOP in der BFRT-Manschette (100) erreicht wurde, bis der Druckwert den ausgewählten Druckwert oder den Prozentsatz des LOP erreicht; und,
i. Trainieren der Extremität des Benutzers, während die BFRT-Manschette (100) an der Extremität des Benutzers positioniert ist; und
wobei A) die Motor-/Pumpeneinheit (200) die Luftpumpe aktiviert, wenn der Druck in der Luftkammer während des Trainingsschritts unter den ausgewählten Druckwert, unter den Prozentsatz des LOP, auf oder unter einen voreingestellten Mindestdruckwert oder auf oder unter einen voreingestellten Mindestprozentsatz des LOP fällt, um den Druck in der Luftkammer so zu erhöhen, dass er innerhalb eines bestimmten Druckbereichs oder innerhalb eines bestimmten Bereichs des LOP-Prozentsatzes liegt, und/oder B) die Motor-/Pumpeneinheit (200) oder die BFRT-Manschette (100) bewirkt, dass der Druck in der Luftkammer verringert wird, wenn der Druck in der Luftkammer den ausgewählten Druckwert überschreitet, den Prozentsatz des LOP überschreitet, einen voreingestellten maximalen Druckwert erreicht oder überschreitet oder einen voreingestellten maximalen Prozentsatz des LOP erreicht oder überschreitet, während des Trainingsschritts , um den Druck in der Luftkammer auf einen bestimmten Druckbereich oder einen bestimmten Bereich des LOP-Prozentsatzes zu senken; und
wobei I) die Motor-/Pumpeneinheit (200) oder die BFRT-Manschette (100) bewirkt, dass der Druck in der Luftkammer unter den ausgewählten Druckwert oder den Prozentsatz des LOP gesenkt wird, wenn die Motor-/Pumpeneinheit (200) oder die BFRT-Manschette (100) nach einer vorgewählten Zeitspanne eine Inaktivität bei der Nutzung der BFRT-Manschette (100) durch den Benutzer feststellt, oder II) die Motor-/Pumpeneinheit (200) oder die BFRT-Manschette (100) bewirkt, dass der Druck in der Luftkammer unter den ausgewählten Druckwert oder den Prozentsatz des LOP gesenkt wird, nachdem die Motor-/Pumpeneinheit oder die BFRT-Manschette (100) erfasst hat, dass eine bestimmte Anzahl von Wiederholungen abgeschlossen wurde oder eine ausgewählte Übung abgeschlossen wurde;
**gekennzeichnet durch**
automatische Auswahl des ausgewählten Druckwerts oder Prozentsatzes des Extremitätenokklusionsdrucks (LOP) auf der Grundlage einer Eingabe durch den Benutzer der Extremität, an der die BFRT-Manschette (100) angebracht ist.

9. Das in Anspruch 8 definierte Verfahren, das den Schritt der drahtlosen Steuerung der Motor-/Pumpeneinheit umfasst.

10. Ein Blutflussrestriktionstrainingssystem (BFRT), umfassend:
a. einer BFRT-Manschette (100), wobei die BFRT-Manschette (100) i) eine Luftkammer, die zumindest teilweise von einem flexiblen Außenmaterial umschlossen ist, und ii) eine Befestigungsvorrichtung (280) umfasst, die so konfiguriert ist, dass sie die BFRT-Manschette an einem Körperglied eines Benutzers befestigt; und
b. eine Motor-/Pumpeneinheit (200), wobei die Motor-/Pumpeneinheit (200) so konfiguriert ist, dass sie die BFRT-Manschette (100) zumindest teilweise aufbläst, wobei die Motor-/Pumpeneinheit (200) ein Gehäuse (210) umfasst, sowie eine Luftverbindungsanordnung (280), die das Gehäuse (210) fluidisch mit der BFRT-Manschette (100) verbindet, um zu ermöglichen, dass gepumpte Luft aus der Motor-/Pumpeneinheit (200) zu der Luftkammer strömt und diese zumindest teilweise aufbläst, wobei das Gehäuse (210) zumindest teilweise i) eine Luftpumpe, ii) eine Energiequelle und iii) eine Steuerung enthält; wobei das Gehäuse eine Größe von weniger als 6,55 × 10³ cm³ (400 Kubikzoll) und ein Gewicht von weniger als 1,81 kg (4 Ibs) aufweist; und
wobei die Steuerung so konfiguriert ist, dass sie a) die Motor-/Pumpeneinheit (200) veranlasst, Luft an die BFRT-Manschette (100) zu liefern, um dadurch zu bewirken, dass sich die BFRT-Manschette (100) aufbläst, bis der LOP der Extremität erreicht ist; b) den Luftstrom von der Motor-/Pumpeneinheit (200) zur BFRT-Manschette (100) zu unterbrechen, nachdem der LOP für die Extremität erreicht wurde, c) den Druck in der BFRT-Manschette (100) zu verringern, nachdem der LOP in der BFRT-Manschette (100) erreicht wurde, bis ein Druckwert in der Luftkammer einen ausgewählten Druckwert oder einen ausgewählten Prozentsatz des LOP erreicht hat; und
wobei die Steuerung so konfiguriert ist, dass sie A) die Motor-/Pumpeneinheit (200) zu aktivieren, wenn der Druck in der Luftkammer unter den ausgewählten Druckwert fällt, unter den ausgewählten Prozentsatz des LOP fällt, auf oder unter einen voreingestellten Mindestdruckwert fällt oder auf oder unter einen voreingestellten Mindestprozentsatz des LOP fällt, um den Druck in der Luftkammer so zu erhöhen, dass er innerhalb eines bestimmten Druckbereichs oder innerhalb eines bestimmten Bereichs des LOP-Prozentsatzes liegt, und/oder B) die Motor-/Pumpeneinheit (200) aktiviert, um zu bewirken, dass der Druck in der Luftkammer verringert wird, wenn der Druck in der Luftkammer den ausgewählten Druckwert überschreitet, den Prozentsatz des LOP überschreitet, einen voreingestellten maximalen Druckwert erreicht oder überschreitet oder einen voreingestellten maximalen LOP-Prozentsatzwert erreicht oder überschreitet, um den Druck in der Luftkammer auf einen bestimmten Druckbereich oder einen bestimmten LOP-Prozentsatzbereich zu senken;
**dadurch gekennzeichnet, dass**
die Steuerung so konfiguriert ist, dass sie den ausgewählten Druckwert oder den Prozentsatz des Limb-Occlusion-Drucks (LOP) automatisch auf der Grundlage einer Eingabe durch den Benutzer der Extremität auswählt, an der die BFRT-Manschette (100) angebracht ist.

11. Das BFRT-System gemäß Anspruch 10, wobei die Steuerung so konfiguriert ist, dass sie I) die Motor-/Pumpeneinheit (200) veranlasst, den Druck in der Luftkammer unter den ausgewählten Druckwert oder den Prozentsatz des LOP zu senken, wenn die Motor-/Pumpeneinheit (200) oder die BFRT-Manschette (100) nach einer vorgewählten Zeitspanne eine Inaktivität bei der Verwendung der BFRT-Manschette feststellt, oder II) die Motor-/Pumpeneinheit (200) veranlasst, den Druck in der Luftkammer unter den ausgewählten Druckwert oder den Prozentsatz des LOP zu senken, wenn die Motor-/Pumpeneinheit (200) oder die BFRT-Manschette (100) feststellt, dass eine bestimmte Anzahl von Wiederholungen abgeschlossen wurde oder eine ausgewählte Übung abgeschlossen wurde.

## Revendications

1. Procédé d'entraînement physique utilisant un système d'entraînement par restriction du flux sanguin (BFRT) (20), comprenant:
a. la fourniture d'un brassard BFRT (100), ledit brassard BFRT (100) comprenant i) un matériau extérieur souple, ii) une chambre à air au moins partiellement encapsulée par ledit matériau extérieur souple, et iii) un dispositif de connexion (280) configuré pour fixer ledit brassard BFRT (100) à un membre d'un utilisateur, ledit matériau extérieur souple ayant une longueur d'au moins 20 cm et une largeur d'au moins 5 cm;
b. fournir une unité moteur/pompe (200), ladite unité moteur/pompe (200) étant configurée pour gonfler au moins partiellement ledit brassard BFRT (100), ladite unité moteur/pompe (200) comprenant un boîtier (210) et un dispositif de raccordement d'air (280) qui relie de manière fluidique ledit boîtier (210) audit brassard BFRT (100) afin de permettre à l'air pompé par ladite unité moteur/pompe (200) de s'écouler vers ladite chambre à air et de la gonfler au moins partiellement, ledit boîtier (210) ayant un volume inférieur à 6,55 x 10³ cm³ (400 pouces cubes) et un poids inférieur à 1,81 kg (4 livres) ; ledit boîtier (210) contenant au moins partiellement i) une pompe à air, ii) une source d'alimentation, et iii) un capteur de pression;
c. placer ledit brassard BFRT (100) sur un membre de l'utilisateur;
d. connecter ledit brassard BFRT (100) à ladite unité moteur/pompe (200) pour permettre la circulation d'air entre ledit brassard BFRT (100) et ladite unité moteur/pompe (200);
e. sélectionner une valeur de pression ou un pourcentage de pression d'occlusion du membre (LOP) en fonction du membre de l'utilisateur sur lequel ledit brassard BFRT (100) est placé, ladite valeur de pression étant inférieure à ladite LOP, ledit pourcentage ne dépassant pas 90 %;
f. amener ladite unité moteur/pompe (200) à fournir de l'air audit brassard BFRT (100) afin de provoquer le gonflage dudit brassard BFRT (100) jusqu'à ce que ladite LOP dudit membre soit atteinte;
g. interrompre le flux d'air provenant de ladite unité moteur/pompe (200) vers ledit brassard BFRT (100) une fois que ladite LOP a été atteinte pour le membre;
h. réduire la pression dans ledit brassard BFRT (100) dans un délai de 0,001 à 30 secondes après l'obtention dudit LOP dans ledit brassard BFRT (100) jusqu'à ce que ladite valeur de pression corresponde à ladite valeur de pression sélectionnée ou audit pourcentage de LOP; et,
i. faire faire de l'exercice au membre de l'utilisateur pendant que ledit brassard BFRT (100) est positionné sur ledit membre dudit utilisateur ; et dans lequel A) ladite unité moteur/pompe active ladite pompe à air lorsque ladite pression dans ladite chambre à air tombe en dessous de ladite valeur de pression sélectionnée, tombe en dessous dudit pourcentage de ladite LOP, tombe à ou en dessous d'une valeur de pression minimale prédéfinie, ou tombe à ou en dessous d'une valeur de pourcentage de LOP minimale prédéfinie pendant ladite étape d'exercice visant à augmenter ladite pression dans ladite chambre à air, et/ou B) ladite unité moteur/pompe (200) ou ledit brassard BFRT (100) provoque une réduction de ladite pression dans ladite chambre à air lorsque ladite pression dans ladite chambre à air dépasse ladite valeur de pression sélectionnée, dépasse ledit pourcentage de ladite LOP, atteint ou dépasse une valeur de pression maximale prédéfinie, ou atteint ou dépasse une valeur de pourcentage de LOP maximale prédéfinie au cours de ladite étape d'exercice, de manière à diminuer ladite pression dans ladite chambre à air;
**caractérisé par**
la sélection automatique de la valeur de pression sélectionnée ou du pourcentage de pression d'occlusion du membre (LOP) sur la base d'une entrée effectuée par ledit utilisateur concernant le membre sur lequel ledit brassard BFRT (100) est placé.

2. Procédé tel que défini dans la revendication 1, dans lequel ladite étape de réduction est au moins partiellement réalisée en amenant ladite unité moteur/pompe (200) à évacuer l'air dudit brassard BFRT (100).

3. Procédé tel que défini dans la revendication 1, comprenant en outre l'étape consistant à retirer ledit brassard BFRT (100) dudit membre après ladite étape d'exercice dudit membre de l'utilisateur.

4. Procédé selon la revendication 2, comprenant en outre l'étape consistant à retirer ledit brassard BFRT (100) dudit membre après ladite étape d'exercice dudit membre de l'utilisateur.

5. Procédé tel que défini dans l'une quelconque des revendications 1 à 4, dans lequel ledit boîtier (210) de ladite unité moteur/pompe (200) fournit trois types d'informations ou plus choisis parmi le groupe comprenant : a) la pression mesurée, b) la puissance ou le niveau de charge de ladite source d'alimentation, c) un symbole d'avertissement lorsqu'un événement d'avertissement quelconque s'est produit, d) le % LOP sélectionné, e) la pression sélectionnée, f) des informations d'instructions sur la manière d'utiliser ladite unité moteur/pompe et/ou le brassard BFRT (100), et g) des options de sélection du bras ou de la jambe pour le brassard BFRT (100).

6. Procédé tel que défini dans l'une quelconque des revendications 1 à 5, comprenant en outre l'étape consistant à mettre en marche ou à alimenter ladite unité moteur/pompe (200), ladite unité moteur/pompe (200) étant configurée pour s'étalonner à la pression ambiante après que ladite unité moteur/pompe (200) a été mise en marche ou alimentée.

7. Procédé tel que défini dans l'une quelconque des revendications 1 à 6, comprenant en outre l'étape consistant à sélectionner le membre sur lequel ledit ensemble moteur/pompe (200) et ledit brassard BFRT (100) doivent être placés.

8. Procédé d'exercice utilisant un système d'entraînement par restriction du flux sanguin (BFRT) comprenant:
a. la fourniture d'un brassard BFRT (100), ledit brassard BFRT (100) comprenant i) une chambre à air au moins partiellement encapsulée par ledit matériau extérieur souple, et ii) un dispositif de connexion configuré pour fixer ledit brassard BFRT (100) au membre d'un utilisateur;
b. fournir une unité moteur/pompe (200), ladite unité moteur/pompe (200) étant configurée pour gonfler au moins partiellement ledit brassard BFRT (100), ladite unité moteur/pompe (200) comprenant un boîtier (210) et un dispositif de raccordement pneumatique (280) qui relie de manière fluidique ledit boîtier (210) audit brassard BFRT (100) pour permettre à l'air pompé provenant de ladite unité moteur/pompe (200) de s'écouler vers ladite chambre à air et de la gonfler au moins partiellement, ledit boîtier (210) contenant au moins partiellement i) une pompe à air, et ii) une source d'alimentation;
c. placer ledit brassard BFRT (100) sur un membre de l'utilisateur;
d. raccorder ledit brassard BFRT (100) à ladite unité moteur/pompe (200) pour permettre la circulation d'air entre ledit brassard BFRT (100) et ladite unité moteur/pompe (200);
e. sélectionner une valeur de pression ou un pourcentage de la pression d'occlusion du membre (LOP), ladite valeur de pression étant inférieure à ladite LOP;
f. amener ladite unité moteur/pompe (200) à fournir de l'air audit brassard BFRT (100) afin de provoquer le gonflage dudit brassard BFRT (100) jusqu'à ce que ladite LOP dudit membre soit atteinte;
g. interrompre le débit d'air provenant dudit ensemble moteur/pompe (200) vers ledit brassard BFRT (100) une fois que ladite pression optimale de gonflage (LOP) a été atteinte pour le membre;
h. réduire la pression dans ledit brassard BFRT (100) après que ledit LOP a été obtenu dans ledit brassard BFRT (100) jusqu'à ce que ladite valeur de pression corresponde à ladite valeur de pression sélectionnée ou audit pourcentage dudit LOP; et,
i. faire faire de l'exercice au membre dudit utilisateur tandis que ledit brassard BFRT (100) est positionné sur ledit membre dudit utilisateur; et
dans lequel A) ladite unité moteur/pompe (200) active ladite pompe à air lorsque ladite pression dans ladite chambre à air tombe en dessous de ladite valeur de pression sélectionnée, tombe en dessous dudit pourcentage de ladite LOP, tombe à ou en dessous d'une valeur de pression minimale prédéfinie, ou tombe à ou en dessous d'une valeur de pourcentage de LOP minimale prédéfinie pendant ladite étape d'exercice de manière à augmenter ladite pression dans ladite chambre à air pour qu'elle se situe dans une certaine plage de pression ou dans une certaine plage de pourcentage de LOP, et/ou B) ladite unité moteur/pompe (200) ou ledit brassard BFRT (100) provoque la réduction de ladite pression dans ladite chambre à air lorsque ladite pression dans ladite chambre à air dépasse ladite valeur de pression sélectionnée, dépasse ledit pourcentage dudit LOP, atteint ou dépasse une valeur de pression maximale prédéfinie, ou atteint ou dépasse une valeur de pourcentage LOP maximale prédéfinie au cours de ladite étape d' de l'exercice, afin de diminuer ladite pression dans ladite chambre à air pour la maintenir dans une certaine plage de pression ou dans une certaine plage de pourcentage LOP; et
dans lequel I) ladite unité moteur/pompe (200) ou ledit brassard BFRT (100) provoque la réduction de ladite pression dans ladite chambre à air en dessous de ladite valeur de pression sélectionnée ou dudit pourcentage dudit LOP lorsque ladite unité moteur/pompe (200) ou ledit brassard BFRT (100) détecte une inactivité dans l'utilisation dudit brassard BFRT (100) par ledit utilisateur après une période de temps présélectionnée, ou II) ladite unité moteur/pompe (200) ou ledit brassard BFRT (100) provoque la réduction de ladite pression dans ladite chambre à air en dessous de ladite valeur de pression sélectionnée ou dudit pourcentage dudit LOP après que ledit ensemble moteur/pompe ou ledit brassard BFRT (100) détecte qu'un certain nombre de répétitions ont été effectuées ou qu'un exercice sélectionné a été terminé;
**caractérisé par**
la sélection automatique de la valeur de pression sélectionnée ou du pourcentage de pression d'occlusion du membre (LOP) sur la base d'une entrée effectuée par ledit utilisateur concernant le membre sur lequel ledit brassard BFRT (100) est placé.

9. Procédé tel que défini dans la revendication 8, comprenant l'étape consistant à commander sans fil ladite unité moteur/pompe.

10. Système d'entraînement par restriction du flux sanguin (BFRT) comprenant:
a. un brassard BFRT (100), ledit brassard BFRT (100) comprenant i) une chambre à air au moins partiellement encapsulée par un matériau extérieur souple, et ii) un dispositif de connexion (280) configuré pour fixer ledit brassard BFRT au membre d'un utilisateur; et
b. un ensemble moteur/pompe (200), ledit ensemble moteur/pompe (200) étant configuré pour gonfler au moins partiellement ledit manchon BFRT (100), ledit ensemble moteur/pompe (200) comprenant un boîtier (210), et un dispositif de raccordement pneumatique (280) qui relie de manière fluidique ledit boîtier (210) audit manchon BFRT (100) afin de permettre à l'air pompé par ladite unité moteur/pompe (200) de s'écouler vers ladite chambre à air et de la gonfler au moins partiellement, ledit boîtier (210) contenant au moins partiellement i) une pompe à air, ii) une source d'alimentation, et iii) un contrôleur ; ledit boîtier ayant une taille inférieure à 6,55 x 10³ cm³ (400 pouces cubes) et un poids inférieur à 1,81 kg (4 livres); et
dans lequel ledit contrôleur est configuré pour a) amener ladite unité moteur/pompe (200) à fournir de l'air audit brassard BFRT (100) afin de provoquer ainsi le gonflement dudit brassard BFRT (100) jusqu'à ce que ladite LOP dudit membre soit obtenue ; b) interrompre le flux d'air provenant de ladite unité moteur/pompe (200) vers ledit brassard BFRT (100) une fois que ledit LOP a été obtenu pour le membre, c) réduire la pression dans ledit brassard BFRT (100) une fois que ledit LOP a été obtenu dans ledit brassard BFRT (100) jusqu'à ce qu'une valeur de pression dans ladite chambre à air atteigne une valeur de pression sélectionnée ou un pourcentage sélectionné dudit LOP; et
dans lequel ledit contrôleur est configuré pour A) activer ladite unité moteur/pompe (200) lorsque ladite pression dans ladite chambre à air tombe en dessous de ladite valeur de pression sélectionnée, tombe en dessous dudit pourcentage sélectionné dudit LOP, tombe à ou en dessous d'une valeur de pression minimale prédéfinie, ou tombe à ou en dessous d'une valeur de pourcentage LOP minimale prédéfinie, de manière à augmenter ladite pression dans ladite chambre à air pour qu'elle se situe dans une certaine plage de pression ou dans une certaine plage de pourcentage LOP, et/ou B) active ladite unité moteur/pompe (200) pour provoquer la réduction de ladite pression dans ladite chambre à air lorsque ladite pression dans ladite chambre à air dépasse ladite valeur de pression sélectionnée, dépasse ledit pourcentage dudit LOP, atteint ou dépasse une valeur de pression maximale prédéfinie, ou atteint ou dépasse une valeur de pourcentage LOP maximale prédéfinie, de manière à réduire ladite pression dans ladite chambre à air pour la maintenir dans une certaine plage de pression ou dans une certaine plage de pourcentage LOP;
**caractérisé en ce que**
ledit contrôleur est configuré pour sélectionner automatiquement ladite valeur de pression sélectionnée ou ledit pourcentage de pression d'occlusion du membre (LOP) sur la base d'une entrée fournie par ledit utilisateur concernant le membre sur lequel ledit brassard BFRT (100) est placé.

11. Système BFRT tel que défini dans la revendication 10, dans lequel ledit contrôleur est configuré pour I) amener ladite unité moteur/pompe (200) à réduire ladite pression dans ladite chambre à air en dessous de ladite valeur de pression sélectionnée ou dudit pourcentage dudit LOP lorsque ladite unité moteur/pompe (200) ou ledit brassard BFRT (100) détecte une inactivité dans l'utilisation dudit brassard BFRT après une période de temps présélectionnée, ou II) amener ladite unité moteur/pompe (200) à réduire ladite pression dans ladite chambre à air en dessous de ladite valeur de pression sélectionnée ou dudit pourcentage dudit LOP lorsque ladite unité moteur/pompe (200) ou ledit brassard BFRT (100) détecte qu'un certain nombre de répétitions ont été effectuées ou qu'un exercice sélectionné a été terminé.
